# EUROPEAN PATENT APPLICATION

(11) **EP 3 906 909 A1**
(43) Date of publication of application: **10.11.2021**
(21) Application number: 20773043.3
(22) Date of filing: 21.02.2020
(51) Int. Cl.: A61F 13/49, A61F 13/51

(54) **DISPOSABLE WEARABLE ARTICLE**

(30) Priority: 18.03.2019 JP 2019050235
(71) Applicant: Daio Paper Corporation, Ehime 799-0492 (JP)
(72) Inventor: TSUNODA, Arika, Sakura-shi, Tochigi 329-1411 (JP)
(74) Representative: Williams Powell
(86) International application number: PCT/JP2020/007035
(87) International publication number: WO 2020/189177

(57) **Abstract**

[Problem] To obscure visual color unevenness of colored nonwoven fabric without requiring coloring of a base sheet.

[Solution] The problem is solved by a disposable wearable article including colored nonwoven fabric 100 having an exposed portion, and base sheet 120 adjacent to the inner side of the colored nonwoven fabric, wherein the colored nonwoven fabric 100 is a composite nonwoven fabric having a plurality of layers laid one on top of another, wherein the colored nonwoven fabric 100 has, in addition to outermost layer 101, colored layer 110 colored in a color of the first lightness other than white, wherein the outermost layer 101 of the colored nonwoven fabric 100 has a color of the second lightness higher than the first lightness, and wherein the external surface of the base sheet 120 has a color of the third lightness higher than the first lightness.

## Description

### Technical Field

The present invention relates to disposable wearable articles having colored nonwoven fabric.

### Background Art

Some disposable wearable articles, such as disposable diapers, are known to have nonwoven fabric constituting the external surface colored in other than white, or have other parts colored, for the purpose of making their appearance closer to underwear (see, e.g., Patent Literatures 1 and 2).

The colored nonwoven fabric colored in other than white, having a white base sheet (nonwoven fabric, film, or the like) overlapping either of its front and back sides and nothing overlapping the other of the sides, has problems of apparent unevenness in color shading (referred simply to color unevenness hereinbelow) and lack of good appearance, when visually observed from the other of the sides.

Such color unevenness result from distinct emergence of color unevenness of the colored nonwoven fabric due to its irregular formation (mass distribution), which emergence is caused by obvious color difference between the colored nonwoven fabric and the white base sheet.

Thus, coloring the base sheet, which is on the back side of the colored nonwoven fabric, in a color close to (with little color difference) the color of the colored nonwoven fabric, for example, is one valid solution, as proposed in Patent Literature 3.

However, use of additional colored members will increase the material costs, so that other solutions are demanded.

### Citation List

### Patent Literature

Patent Literature 1: JP 2016-73578 A
Patent Literature 2: JP 2012-170577 A
Patent Literature 3: JP 2017-225508 A

### Summary of Invention

### Technical Problem

In view of the above, it is a primary object of the present invention to obscure the visual color unevenness of colored nonwoven fabric by means other than coloring of a base sheet.

### Solution to Problem

Typical aspects of the present invention solving the above problem are as follows.

### <First Aspect>

A disposable wearable article including:
colored nonwoven fabric having an exposed portion, and
a base sheet adjacent to an inner side of the colored nonwoven fabric,
wherein the colored nonwoven fabric is a composite nonwoven fabric having a plurality of layers piled one on top of another,
wherein the colored nonwoven fabric has, in addition to an outermost layer, a colored layer colored in a color of a first lightness other than white,
wherein the outermost layer of the colored nonwoven fabric has a color of a second lightness higher than the first lightness, and
wherein an external surface of the base sheet has a color of a third lightness higher than the first lightness.

### (Function and Effect)

The disposable wearable article of the invention is characterized in that the colored nonwoven fabric is a composite nonwoven fabric, and has, in addition to the outermost layer, a colored layer colored in a color of the first lightness other than white, and the outermost layer has a color of the second lightness higher than the first lightness. In this case, in visual observation of the colored nonwoven fabric from outside, the colored layer is observed through the outermost layer, and thus the color of the colored layer is observed as being thinned (lightened). In addition, the outermost layer naturally has irregular formation, which causes unevenness in the thinning degree of the visually observed color of the colored layer. As a result, not only the visual color of the colored layer is thinned, but also the color unevenness thereof is made finer, so that the color unevenness of the colored layer is unexpectedly obscured even when the external surface of the base sheet is in a color of the lightness higher than the first lightness (i.e., the color unevenness of the colored nonwoven fabric is apparent). Consequently, the visual color unevenness of the colored nonwoven fabric may be obscured irrespective of whether the base sheet is colored or not.

### <Second Aspect>

The disposable wearable article according to the first embodiment, wherein the color of the first lightness other than white has a CIELAB L* value of 20 to 90, and an absolute value of at least one of a* and b* values is 0 to 40, wherein the color of the second lightness and the color of the third lightness each has a CIELAB L* value of 60 to 100, and wherein a color difference ΔE between the colors of the second and the third lightness is 30 or less.

### (Function and Effect)

The colors of the colored layer and the outermost layer of the colored nonwoven fabric as well as of the base sheet are not particularly limited, and are preferably within the ranges of the second aspect of the invention. When the color of the outermost layer of the colored nonwoven fabric and the color of the base sheet is close, color unevenness preventive effect is particularly great.

### <Third Aspect>

The disposable wearable article according to the first or second aspect, wherein the colored nonwoven fabric is a composite nonwoven fabric of 2 to 4 layers having a fineness of 1.5 to 5.0 dtex and a basis weight of 10 to 20 g/m².

### (Function and Effect)

In order to impart soft texture, the colored nonwoven fabric preferably falls within the above-mentioned ranges. In this case, however, formation of the nonwoven fabric tends to appear as color unevenness. Irrespective of this, with the above-mentioned color unevenness preventive structure, little color unevenness as well as the soft texture is imparted to the colored nonwoven fabric.

### <Fourth Aspect>

The disposable wearable article according to any one of the first to third aspects,
wherein the disposable wearable article has an elastic sheet stretchable structure including a first sheet layer, a second sheet layer facing to the first sheet layer and exposed to an external surface of the article, and an elastic sheet laid between the first and second layers, wherein the first and second layers are joined through joining holes penetrating the elastic sheet at multiple joining portions arranged at intervals,
the colored nonwoven fabric is the second sheet layer, and
the base sheet is the elastic sheet.

### (Function and Effect)

In the elastic sheet stretchable structure as in the disposable wearable article of this aspect, it is preferred that the colored nonwoven fabric and the base sheet have the above-mentioned structures. In particular, with this elastic sheet stretchable structure, the joining holes in the elastic sheet are visually observable through the colored nonwoven fabric, and may thus adversely affect the appearance. For example, some people may see the joining holes of different sizes as visually non-uniform pattern, like color unevenness. However, with the above-mentioned color unevenness preventive structure, not only the color unevenness of the colored nonwoven fabric but also the joining holes may be obscured.

### <Fifth Aspect>

The disposable wearable article according to any one of the first to fourth aspects, further including an absorbent body, a liquid impervious sheet covering a back surface side of the absorbent body, and outer nonwoven fabric covering a back surface side of the liquid impervious sheet,
wherein the colored nonwoven fabric is the outer nonwoven fabric, and
wherein the base sheet is the liquid impervious sheet.

### (Function and Effect)

In disposable wearable articles having an absorbent body, such as disposable diapers, the back surface side of the absorbent body is usually covered with a liquid impervious sheet, such as a waterproof film. Further, for imparting fabric-like appearance and texture to the external surface of the articles, it is also popular to cover the back surface side of the liquid impervious sheet with outer nonwoven fabric. Thus, in providing such disposable wearable articles with coloring, colored nonwoven fabric is preferably used as the outer nonwoven fabric and, in this case, it is preferred to adopt the color unevenness preventive structure discussed above.

### Effect of the invention

According to the present invention, visual color unevenness of the colored nonwoven fabric may be obscured by means other than coloring of the base sheet.

### Brief Description of Drawings

Fig. 1 is a plan view of the underpants-type disposable diaper in the spread state (internal surface side).
Fig. 2 is a plan view of the underpants-type disposable diaper in the spread state (external surface side).
Fig. 3 is a plan view of the underpants-type disposable diaper in the spread state, showing only the relevant parts.
Fig. 4(a) is a sectional view taken along lines C-C in Fig. 1, and Fig. 4(b) is a sectional view taken along lines E-E in Fig. 1.
Fig. 5 is a cross-sectional view taken along lines A-A in Fig. 1.
Fig. 6 is a cross-sectional view taken along lines B-B in Fig. 1.
Fig. 7 is a plan view of the relevant part of the stretchable region of the underpants-type disposable diaper in the spread state (internal surface side).
Fig. 8(a) is a sectional view corresponding to lines C-C in Fig. 7, and Fig. 8(b) is a sectional view corresponding to lines E-E in Fig. 7.
Fig. 9 shows plan and cross-sectional views illustrating an exemplary arrangement of the joining portions.
Fig. 10 shows plan views illustrating exemplary arrangements of the joining portions.
Fig. 11 illustrates an exemplary arrangement of the joining portions, wherein Fig. 11(a) is a plan view and Fig. 11(b) is a cross-sectional view taken along lines B-B in Fig. 11(a).
Fig. 12 illustrates an exemplary arrangement of the joining portions, wherein Fig. 12(a) is a plan view and Fig. 12(b) is a cross-sectional view taken along lines B-B in Fig. 12(a).
Fig. 13 is a plan view illustrating an exemplary arrangement of the joining portions.
Fig. 14 is a plan view illustrating an exemplary arrangement of the joining portions.
Fig. 15 is a plan view illustrating an exemplary arrangement of the joining portions.
Fig. 16 is a plan view illustrating an exemplary arrangement of the joining portions.
Fig. 17 is a plan view illustrating an exemplary arrangement of the joining portions.
Fig. 18 is a plan view illustrating an exemplary arrangement of the joining portions.
Fig. 19 is a plan view illustrating an exemplary arrangement of the joining portions.
Fig. 20 illustrates explanatory views of exemplary shapes of the joining portions.
Fig. 21 shows cross-sectional views illustrating exemplary joining structures at the joining portions.
Fig. 22 shows cross-sectional views illustrating exemplary joining structures at the joining portions.
Fig. 23 shows plan views illustrating exemplary joining structures.
Fig. 24 is a schematic view of ultrasonic sealing apparatus.
Fig. 25 shows comparative explanatory views of the exemplary arrangements of the joining portions.
Fig. 26 is a plan view of the underpants-type disposable diaper in the spread state (internal surface side).
Fig. 27(a) is a sectional view taken along lines C-C in Fig. 26, and Fig. 27(b) is a sectional view taken along lines E-E in Fig. 26.
Fig. 28 is a plan view of an underpants-type disposable diaper in the spread state (external surface side).
Fig 29 shows cross-sectional views illustrating the layered structures of the colored nonwoven fabric and the base sheet.
Fig. 30 shows cross-sectional views illustrating the layered structures of the colored nonwoven fabric and the base sheet.

### Description of Embodiments

Embodiments of the disposable wearable articles will now be described in detail with reference to the accompanying drawings. In the drawings, dotted pattern regions represent adhesives as joining means for joining the components on the front or back surface side of the respective regions, and may be formed by, for example, solid, bead, curtain, summit, or spiral application, or pattern coating (transfer of a hot melt adhesive by relief printing) of a hot melt adhesive, and fixed portions of elastic member may be formed, in place of or in addition to this, by application of a hot melt adhesive to the external surface of the elastic member with a comb gun or a surewrap. Examples of the hot melt adhesive include, but not limited to, EVA-based, adherent rubber-based (elastomer-based), polyolefin-based, and polyester/polyamide-based adhesives. The joining means for joining the components may alternatively be material welding, such as heat sealing or ultrasonic sealing.

As the nonwoven fabric in the description hereinbelow, commonly known nonwoven fabric may suitably be used depending on the sites or purposes. Examples of the constituent fibers of the nonwoven fabric include, but not limited to, synthetic fibers, such as polyolefin-based, e.g., polyethylene or polypropylene, polyester-based, or polyamide-based fibers (including not only single component fibers, but also composite fibers, such as of core/sheath type), as well as regenerated fibers, such as rayon or cuprammonium, or natural fibers, such as cotton, and also mixtures thereof. For improved flexibility of the nonwoven fabric, the constituent fibers may preferably be crimped fibers. The constituent fibers of the nonwoven fabric may also be hydrophilic fibers (including those rendered hydrophilic with hydrophilizers), hydrophobic fibers, or water-repelling fibers (including those rendered water-repelling with water repellents). Further, nonwoven fabric may generally be categorized into short fiber nonwoven, long fiber nonwoven, spunbond nonwoven, melt blown nonwoven, spunlace nonwoven, thermal bonded (air through) nonwoven, needle-punched nonwoven, point-bonded nonwoven, composite nonwoven (SSS nonwoven fabric having the same or similar nonwoven layers laid one on top of another, as well as SMS or SMMS nonwoven fabric having different nonwoven layers laid one on top of another, i.e., melt blown layer placed between spunbond layers), or the like nonwoven fabric, depending on the length of the fibers, method of forming the sheet, method of joining the fibers, or layered structure, and any of these nonwoven fabric may be used. The composite nonwoven fabric refers to those having all the layers integrally manufactured and subjected to fiber joining process all over the layers, and does not include those having a plurality of nonwoven fabric layers separately manufactured and bonded with joining means, such as hot melt adhesives.

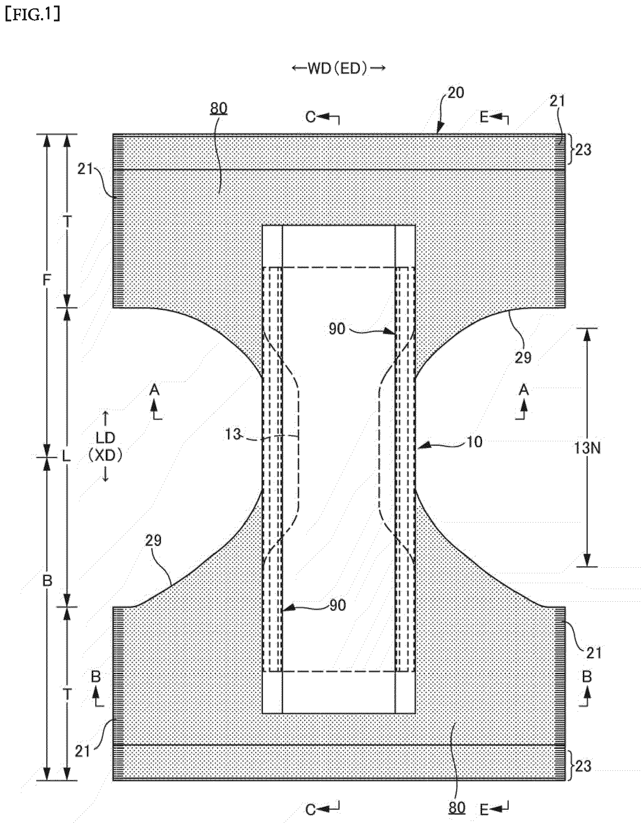

Figs. 1 to 6 show an example of the underpants-type disposable diaper (sometimes referred simply to diaper hereinbelow). Reference sign ED denotes the stretchable direction ED of the stretchable regions, which direction is the same as the width direction WD of the diaper in the present embodiment. Reference sign XD denotes the direction orthogonal to the stretchable direction ED, which direction is the same as the front-back direction LD of the diaper in the present embodiment.

This underpants-type disposable diaper has outer member 20 forming front body F and back body B and inner member 10 integrally fixed on the internal surface side of the outer member 20, and the inner member 10 has liquid pervious top sheet 11, liquid impervious sheet 12, and absorbent body 13 interposed therebetween. In production, the back surface of the inner member 10 is joined to the internal surface (upper surface) of the outer member 20 by joining means, such as a hot melt adhesive, then the inner and outer members 10, 20 are folded along the center of the front-back direction LD (longitudinal direction), which center is the boundary between the front body F and the back body B, and each side portion of the front body F and each side portion of the back body B are joined together by means of thermal welding or a hot melt adhesive to form side seal portion 21, to thereby obtain an underpants-type disposable diaper having a waist opening and a pair of right and left leg openings thus formed.

### (Structural Example of Inner Member)

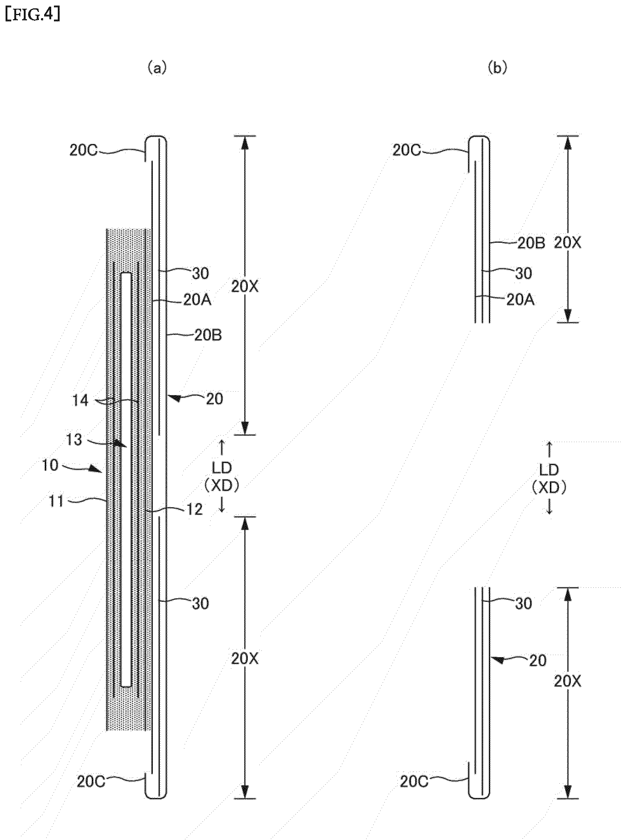

The inner member 10 has a structure, as shown in Figs. 4 to 6, including liquid pervious top sheet 11, liquid impervious sheet 12 made of, for example, polyethylene, and absorbent body 13 interposed therebetween which absorbs and holds excrement, to absorb and hold excrement liquid passing through the top sheet 11. The planar shape of the inner member 10 is not particularly limited, and may generally be approximately rectangular, as shown in Fig. 1.

As the top sheet 11 covering the front surface side (skin side) of the absorbent body 13, perforated or imperforate nonwoven fabric or porous plastic sheets may preferably be used. With the top sheet 11 having multiple pores formed therethrough, urine or the like is promptly absorbed, providing excellent dry touch property. The top sheet 11 is folded over and around the side edges of the absorbent body 13 and extends to the back surface side of the absorbent body 13.

As the liquid impervious sheet 12 covering the back surface side (non-skin-contacting side) of the absorbent body 13, liquid impervious plastic sheets, such as of polyethylene or polypropylene, may be used, and recently those having moisture permeability are preferably used for preventing stuffiness. Such water-impervious moisturepermeable sheets may be microporous sheets obtained by, for example, melting and kneading an inorganic filler in a polyolefin resin, such as polyethylene or polypropylene, forming the kneaded mixture into a sheet, and then uniaxially or biaxially drawing the sheet.

The absorbent body 13 may be a conventional one, for example, accumulated pulp fibers, an assembly of filaments, such as of cellulose acetate, or nonwoven fabric, to which super absorbent polymers are optionally admixed or fixed. The absorbent body 13 may optionally be wrapped in liquidpervious liquid-holding wrapping sheet 14, such as crepe paper, for maintaining its shape and the polymer therein.

The absorbent body 13 is formed generally in an hourglass shape having, in the crotch portion, narrower portion 13N with a width narrower than those of the front and back side portions. The dimensions of the narrower portion 13N may suitably be decided; its length in the front-back direction may be about 20 to 50% of the entire length of the diaper, and the width of its narrowest portion may be about 40 to 60% of the entire width of the absorbent body 13. With such a narrower portion 13N, and the planar shape of the inner member 10 being substantially rectangular, no-absorbent side portions 17 without the absorbent body 13 are formed in the portions of the inner member 10 corresponding to the narrower portion 13N of the absorbent body 13.

The liquid impervious sheet 12, together with the top sheet 11, is folded back onto the back surface side on both sides of the width of the absorbent body 13. As the liquid impervious sheet 12, an opaque sheet may preferably be used so as not to show the brown color of feces and urine. For opacification, pigments or fillers, such as calcium carbonate, titanium oxide, zinc oxide, white carbon, clay, talc, and barium sulfate, are internally added to plastics, which is formed into films and preferably used.

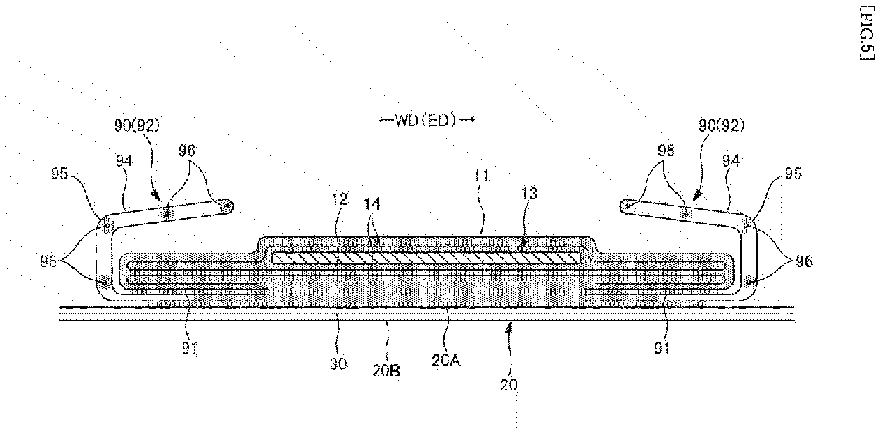

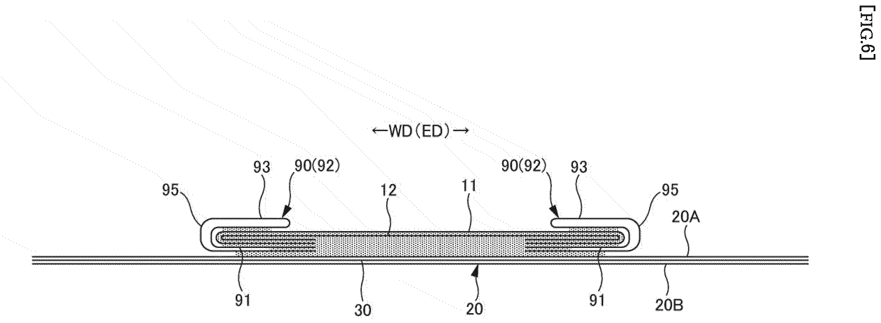

On each side portion of the inner member 10, three-dimensional gather 90 is formed that fits on and around the leg. Each three-dimensional gather 90 has, as shown in Figs. 5 and 6, fixed portion 91 fixed to the corresponding side portion of the back surface of the inner member 10, main body portion 92 extending from the fixed portion 91 through the side of the inner member 10 to above the corresponding side portion of the front surface of the inner member 10, laid-down portions 93 formed by each of the front and back end portions of the main body portion 92 fixed in a laid-down state to the corresponding side portion of the front surface of the inner member 10 (top sheet 11 in the illustrated embodiment), and free portion 94 formed between the laid-down portions 93 without the fixing. Each of these portions is formed of gather sheets 95, which are duplicate sheets formed by folding a sheet, such as of non-woven fabric. The gather sheets 95 are fixed to the inner member 10 all along its front-back direction, with the laid-down portions 93 being arranged forward and backward of the no-absorbent side portion 17, and the free portion 94 extending to the front and back sides of the no-absorbent side portion 17. Between the duplicate gather sheets 95, elongate gather elastic members 96 are arranged at positions including the tip of the free portion. In the finished product, as shown in Fig. 5, the gather elastic members 96 function to raise the free portions 94 by means of their elastic contraction force.

According to the embodiment as shown in Figs. 5 and 6, in the portions other than the laid-down portions 93, the gather elastic members 96 are adhered and fixed to the gather sheets 95 with a hot melt adhesive arranged at the positions of the gather elastic members 96, and the facing surfaces of the gather sheets 95 are adhered to each other, whereas in the laid-down portions 93, no hot melt adhesive is arranged at the positions of the gather elastic members 96, so that the gather elastic members 96 are not adhered to the gather sheets 95, and the facing surfaces of the gather sheets 95 are not adhered to each other at the positions of the gather elastic members 96.

In the embodiment of the three-dimensional gather 90 as shown in Figs. 5 and 6, the main body portion 92 is not folded back.

The gather elastic members 96 may be of a generally used material, such as styrene rubber, polyolefin rubber, urethane rubber, ester rubber, polyurethane, polyethylene, polystyrene, styrene-butadiene, silicone, or polyester. Further, it is preferred, for not being observed easily from outside, that the gather elastic members 96 have a thickness of 925 dtex or less and are arranged at a tension of 150 to 350% and at intervals of 7.0 mm or less. In addition, the gather elastic members 96 may be in the form of strings as shown in the drawings, or in the form of tapes having a certain width.

The material of the gather sheets 95 is not particularly limited, and is preferably water-repelling nonwoven fabric with a coating of a silicone-, paraffinmetal-, or alkyl chromic chloride-based water repelling agent for preventing permeation of urine or the like as well as for preventing rash and improving a touch to the skin (dryness).

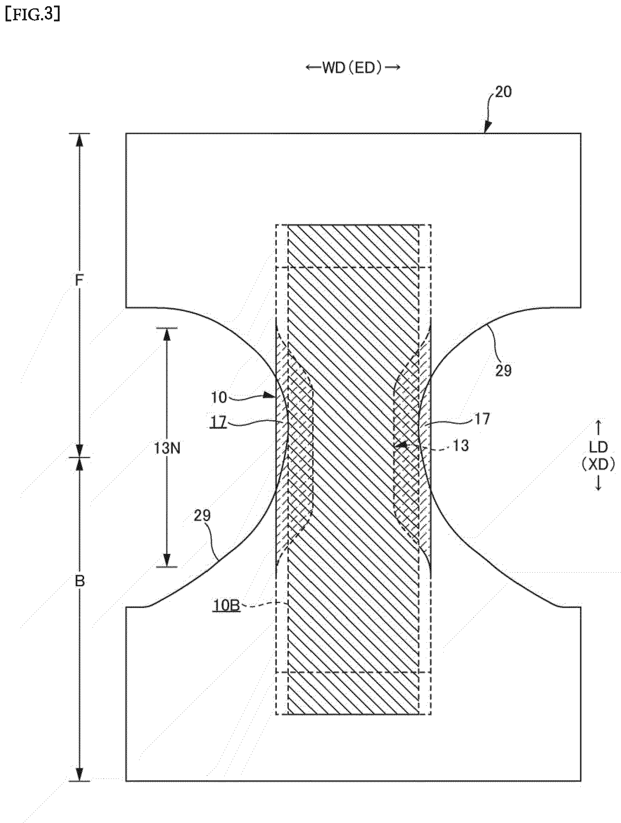

As shown in Figs. 3 to 6, the inner member 10 is bonded on its back surface to the internal surface of the outer member 20 in inner-outer fixed region 10B (hatched region) with a hot melt adhesive or the like. The inner-outer fixed region 10B may suitably be decided, and may cover almost all of the width direction WD of the inner member 10, but preferably is not fixed to the outer member 20 on both ends thereof in the width direction.

### (Structural Example of Outer Member)

In the crotch portion, the side edge portions of the outer member 20 may be located either closer to the center in the width direction than the side edge portions of the inner member 10 as shown in the drawings, or further outward in the width direction than the side edge portions of the inner member 10. The outer member 20 has lower torso portions T, which are the regions in the front-back direction corresponding to the side seal portions 21, and intermediate portion L extending in the front-back direction and arranged between the lower torso portion T of the front body F and the lower torso portion T of the back body B. The illustrated planar shape of the outer member 20 is generally an hourglass-like shape and narrowed to form a leg opening with each side edge of the width of the intermediate portion L, but is not limited to this shape. The outer member 20 may be formed of separate front body F and back body B, which may be arranged at a distance in the front-back direction LD of the diaper in the crotch portion.

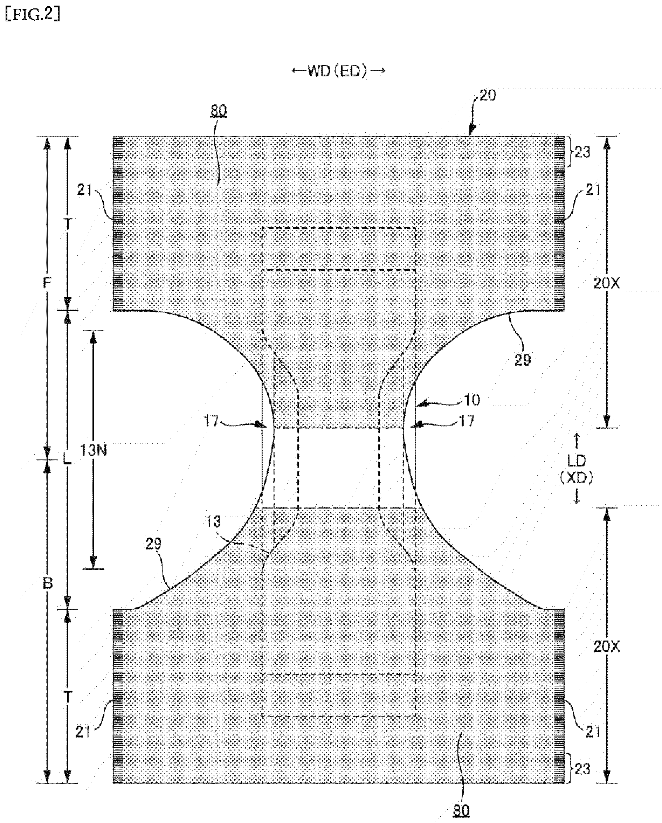

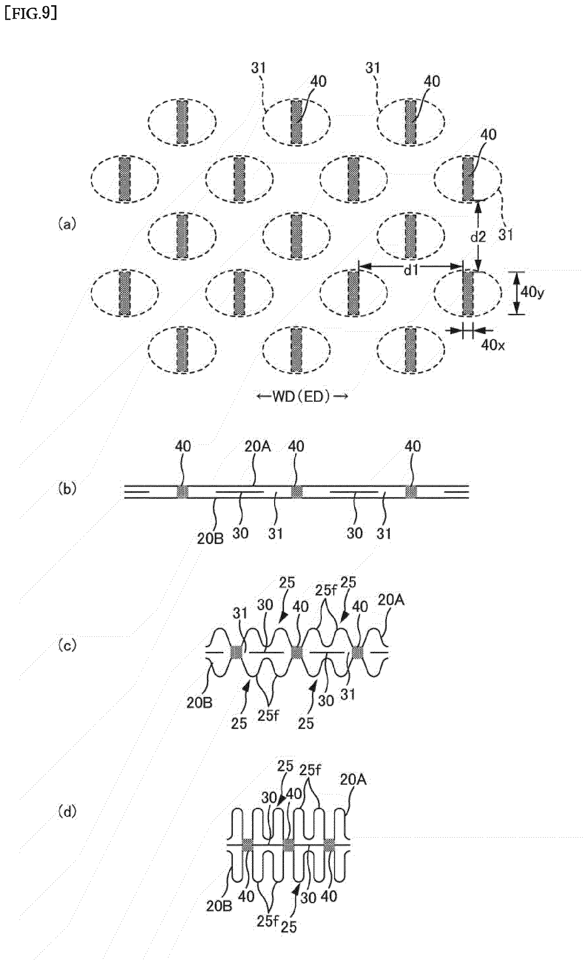

The illustrated outer member 20, except for the middle region in the front-back direction of the intermediate portion L thereof, has elastic sheet stretchable structure 20X wherein elastic sheet 30, such as an elastic film, is interposed between first sheet layer 20A and second sheet layer 20B as shown in Fig. 2 and Figs. 4 to 6, and the first sheet layer 20A and the second sheet layer 20B are joined through joining holes 31 penetrating the elastic sheet 30 at multiple joining portions 40 arranged at intervals as shown in Fig. 9. Here, the stretchable direction ED is the width direction WD of the diaper. The first sheet layer 20A and the second sheet layer 20B may not be joined through the joining holes 31 in the elastic sheet 30, but may indirectly be joined via the elastic sheet 30.

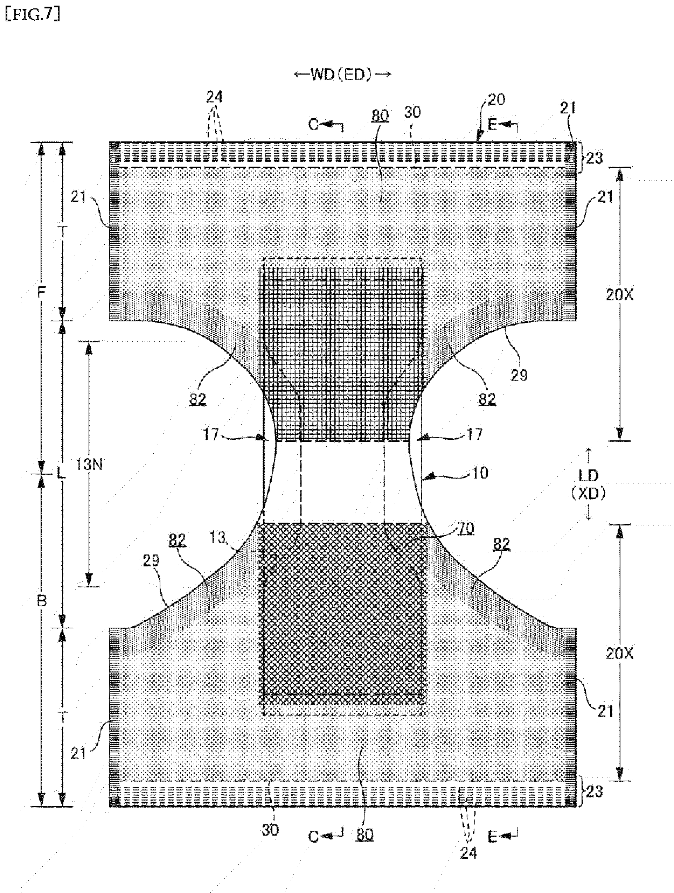

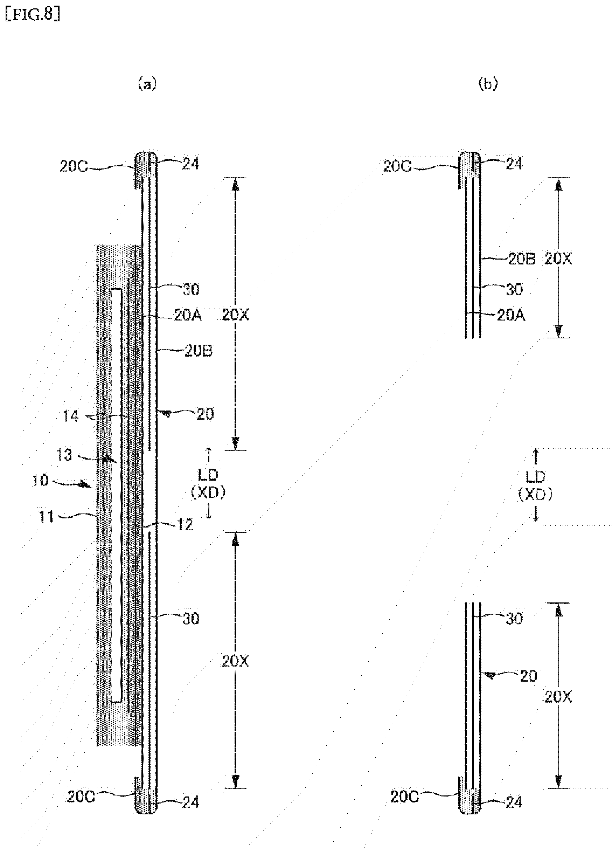

In the embodiment shown in Figs. 1 and 2, the elastic sheet stretchable structure 20X is extended to waist end portions 23. Since adoption of the elastic sheet stretchable structure 20X to the waist end portions 23 may result in insufficient tightening at the waist end portions 23, as necessary, the waist end portions 23 may be provided with a stretchable structure of conventional elongated waist portion elastic members 24, without the elastic sheet stretchable structure 20X, as shown in Figs. 7 and 8. The waist portion elastic members 24 are elongate elastic members, such as a plurality of rubber threads, arranged at intervals in the front-back direction LD, and provide stretchable force to constrict the torso of the body. The waist portion elastic members 24 are not arranged substantially as one bundle with little intervals, but three or more, preferably five or more of them are arranged at intervals of about 3 to 8 mm in the front-back direction to form a prescribed stretchable zone. The stretch rate of the waist portion elastic members 24 when fixed may suitably be decided, and may be about 230 to 320% for ordinary adults. As the waist portion elastic members 24, rubber threads are used in the embodiment shown in the drawings, but other elongate elastic members, such as flat rubber bands, may also be used. Though not shown in the drawings, the waist end portions 23 may be provided with the elastic sheet 30 and also the elongate waist portion elastic members 24 at the locations overlapping the elastic sheet 30, to thereby provide a stretchable structure employing both of the elastic members. Further, in the embodiment shown in the drawings, the edge portions of the leg openings of the outer member 20 are not provided with elongate elastic members extending along the leg openings, but these portions may be provided with elongate elastic members at the locations overlapping the elastic sheet 30, or in place of the elastic sheet 30 in these portions.

Alternatively, though not shown in the drawings, appropriate modifications may be made; for example, the elastic sheet stretchable structure 20X is not provided in the intermediate portion L between the lower torso portion T of the front body F and the lower torso portion T of the back body B; the elastic sheet stretchable structure 20X is provided continuously in the front-back direction LD from the lower torso portion T of the front body F through the intermediate portion L to the lower torso portion T of the back body B; or the elastic sheet stretchable structure 20X is provided only either in the front body F or the back body B.

### (Cover Sheet)

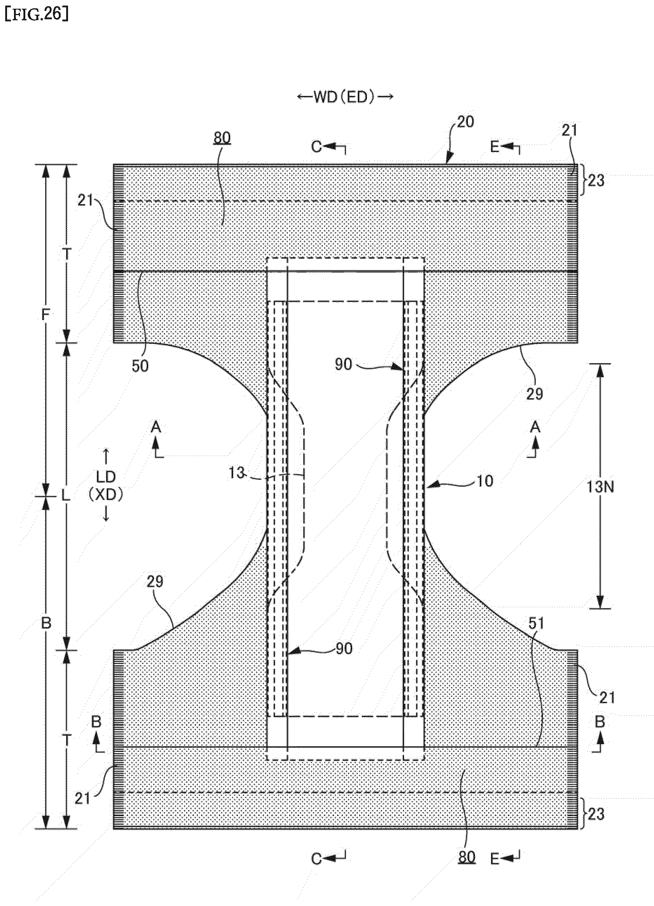

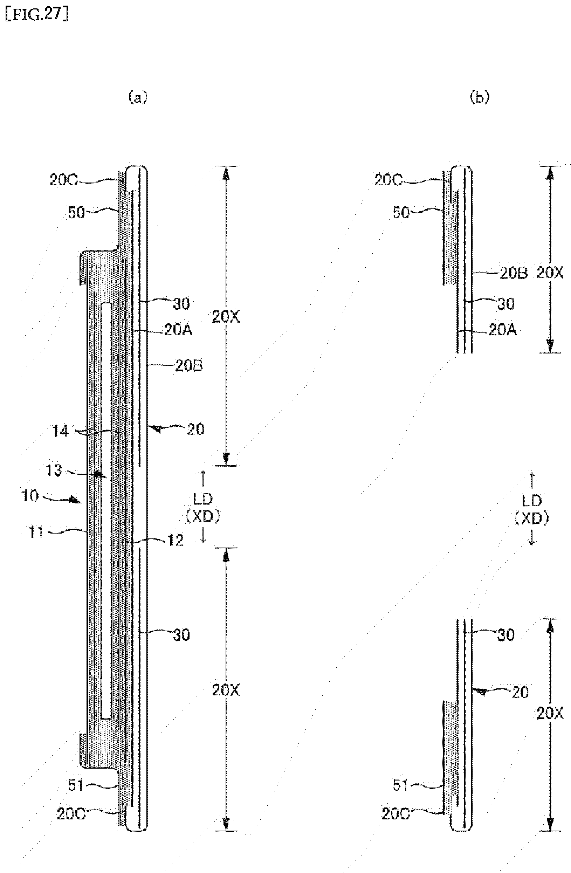

As shown in Figs. 26 and 27, for the purpose of reinforcing the outer member 20 or covering the front and back end portions of the inner member 10 attached to the internal surface of the outer member 20, cover sheets 50, 51 may be provided. Explaining the embodiment shown in the drawings in more detail, the front cover sheet 50 extends on the internal surface of the front body F from the internal surface of waist folded portion 20C to the location overlapping the front end portion of the inner body 10 all over the width direction WD, whereas the back cover sheet 51 extends on the internal surface of the back body B from the internal surface of the waist folded portion 20C to the location overlapping the back end portion of the inner body 10 all over the width direction WD.

The cover sheets 50, 51, formed as separate members and attached as in the illustrated embodiment, have the advantage of high flexibility in material choice, but also the disadvantage of increased numbers of materials and fabrication process. In view of this, the folded portion 20C formed by folding the outer member 20 onto the internal surface of the diaper may be extended to the location overlapping the inner member 10, to thereby form the portions (not shown) equivalent to the cover sheets 50, 51 discussed above.

### (Stretchable Region)

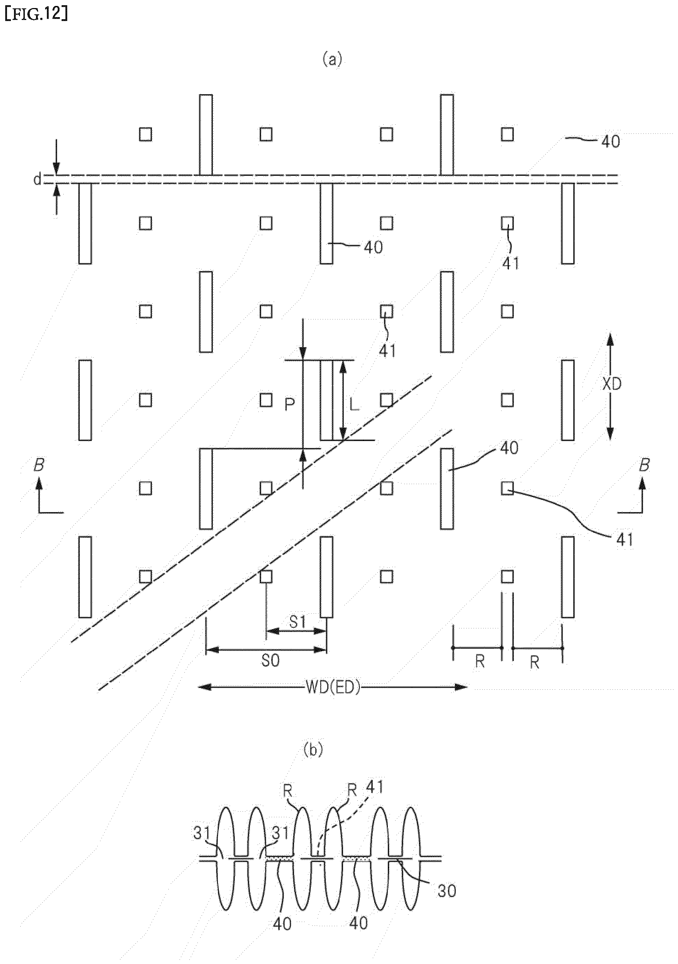

The regions of the outer member 20 having the elastic sheet stretchable structure 20X have the regions stretchable in the width direction WD. The stretchable regions 80 have portions 32 wherein the elastic sheet 30 is linearly continuous along the width direction WD (see Fig. 12(a)), and are contracted in the width direction WD due to the contraction force of the elastic sheet 30 and stretchable in the width direction WD. More specifically, such stretchability may be imparted by means of formation of the elastic sheet stretchable structure 20X by, with the elastic sheet 30 being stretched in the width direction WD, joining of the first sheet layer 20A and the second sheet layer 20B through the joining holes 31 in the elastic sheet 30 at intervals in the width direction WD and in the front-back direction LD orthogonal thereto (direction LD orthogonal to the stretchable direction) to thereby form multiple joining portions 40, and by arranging the joining holes 31 such that the stretchable regions 80 have the portions 32 wherein the elastic sheet 30 is linearly continuous along the width direction WD (see Fig. 12(a)).

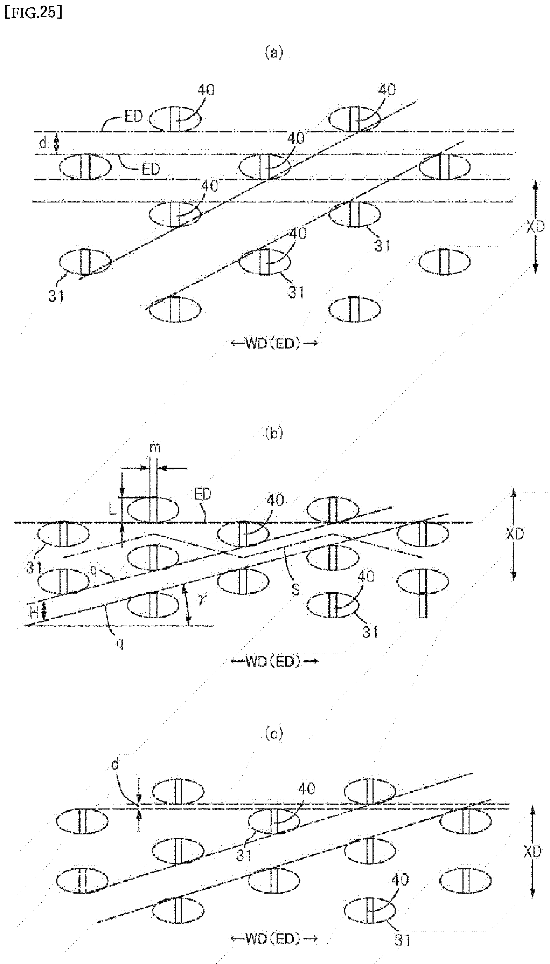

The stretchable regions 80 may have the portions wherein the elastic sheet 30 is linearly continuous along the width direction WD (spaced intervals d to be discussed later) as in the embodiment shown in Fig. 25(a) to be discussed later, or may not have such portions as in the embodiment shown in Fig. 25(b).

In the stretchable regions 80 in the natural length state, the first sheet layer 20A and the second sheet layer 20B between the joining portions 40 are puffed in the directions away from each other to form ridges 25F each extending in the front-back direction LD as shown in Figs. 9 and 12(b), and in the worn state in which the stretchable regions 80 are stretched to some extent in the width direction WD, the ridges 25F are stretched but remain. Further, in the embodiment shown in the drawings, the first sheet layer 20A and the second sheet layer 20B are not joined with the elastic sheet 30 except for at least the first sheet layer 20A and the second sheet layer 20B at the joining portions 40, as can be seen from Fig. 9(c) simulating the worn state and Fig. 9(a) simulating the spread state of the first sheet layer 20A and the second sheet layer 20B. In such states, gaps are formed between each joining hole 31 in the elastic sheet 30 and each joining portion 40, and these gaps impart air-permeability even when the elastic sheet 30 is made of imperforate film or sheet. On the other hand, in the natural length state, the joining holes 31 are narrowed due to further contraction of the elastic sheet 30, resulting in little gaps between each joining hole 31 and each joining portion 40.

The stretchable regions 80 preferably have an elongation at elastic limit in the width direction WD of 190% or more (preferably 225 to 285%). The elongation at elastic limit of the stretchable regions 80 is lowered from the base stretch rate of the as-manufactured elastic sheet 30, by the factors that limit contraction in the width direction WD. Usually, the length L of the joining portions 40 is related to the area rate of the joining portions 40, so that the elongation at elastic limit of the stretchable regions 80 may be adjusted by the area rate of the joining portions 40.

When the stretchable regions 80 have the portions wherein the elastic sheet 30 is linearly continuous along the width direction WD (spaced intervals d to be discussed later) as in the embodiment shown in Fig. 25(a) to be discussed later, the stretching stress of the stretchable regions 80 may be adjusted generally by the sum of the orthogonal dimensions of the portions wherein the elastic sheet 30 is linearly continuous along the width direction WD (equivalent to the spaced intervals d between the joining holes 31). In contrast, when the stretchable regions 80 have no portions wherein the elastic sheet 30 is linearly continuous along the width direction WD as in the embodiment shown in Fig. 25(b), the stretching stress may be adjusted by angle γ to be discussed later, and usually the angle γ is preferably in the range of larger than 0 degree and not larger than 45 degrees, particularly in the range of 10 to 30 degrees.

The area rate of the joining portions 40 and the area of each joining portion 40 in the stretchable regions 80 may suitably be decided, and the following ranges are usually preferred:
Area of each joining portion 40: 0.14 to 3.5 mm² (particularly 0.14 to 1.0 mm²)
Area rate of joining portions 40: 1.8 to 19.1% (particularly 1.8 to 10.6%).

In this way, since the elongation at elastic limit and the stretching stress of the stretchable regions 80 may be adjusted by the areas of the joining portions 40, the stretchable regions 80 may be provided with a plurality of sections with different area rates of the joining portions 40 as shown in Fig. 7, to vary fitting at different sites.

The embodiment shown in Fig. 7 is provided with edge stretchable portions 82 along the leg openings, which portions 82 are made to have a higher area rate of the joining portions 40 compared to the remaining regions, and thus have a lower stretching stress and stretch and contract flexibly.

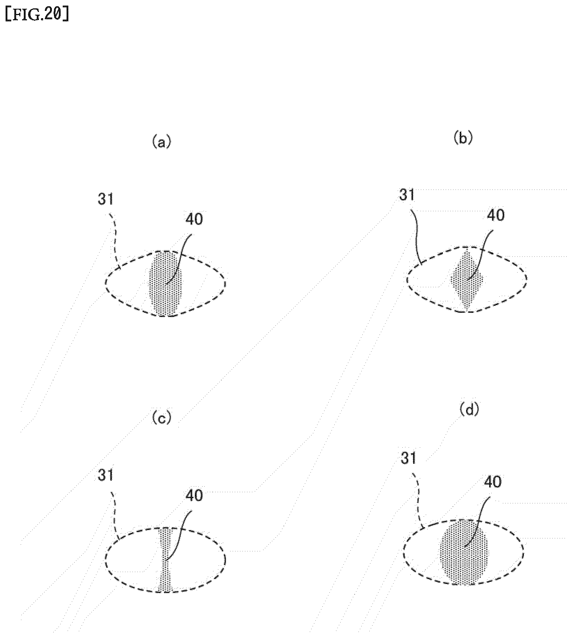

The shapes of each joining portion 40 and each joining hole 31 in the natural length state may suitably be decided, and may be an arbitrary shape, for example, exact circle, ellipse (see Fig. 20(d)), polygon, such as triangle, rectangle (see Fig. 9 and the like), or diamond (see Fig. 20(b)), or a convex lens shape (see Fig. 20(a)), a concave lens shape (see Fig. 20(c)), a star shape, or a cloud shape. The dimensions of each joining portion 40 is not particularly limited, and its maximum length 40y (substantially equal to orthogonal dimension 31y of joining hole 31) is preferably 0.5 to 3.0 mm, particularly 0.7 to 1.1 mm, while its maximum width 40x is preferably 0.1 to 3.0 mm, and particularly 0.1 to 1.1 mm when the shape is elongated in the direction XD orthogonal to the stretchable direction.

The joining holes 31 are associated with mainly the shape of the joining portions 40 (41, 42, 43), manufacturing process, or degree of stretching and contraction.

Examples of layout of the joining portions preferred for the stretching regions will now be described in order hereinbelow, which do not intend to limit the invention.

### (Layout Example 1 of Joining Portions)

Fig. 9 is a typical example disclosed in Patent Literature 1. A group of joining portions 40 are laid out in a staggered arrangement, wherein each joining portion 40 is formed to be elongate in the direction orthogonal to the stretchable direction, and symmetric with respect to the center line passing the center of the stretchable direction (bilaterally symmetric in Fig. 9(a)). Various dimensions may suitably be decided; preferably, the width 40x in the stretchable direction of each joining portion 40 is 0.2 to 0.4 mm, the interval d1 between the joining portions 40 adjacent in the stretchable direction is 3 to 12.9 mm, particularly 5 to 6.4 mm, and the interval d2 between the joining portions 40 adjacent in the direction orthogonal to the stretchable direction is 2 to 10.5 mm, particularly 2.3 to 4.6 mm. Likewise, the length 40y in the direction orthogonal to the stretchable direction of each joining portion 40 is preferably 0.4 to 3.2 mm, particularly 0.7 to 1.4 mm.

As such, the joining portions 40 with a remarkably narrow width 40x in the stretchable direction are laid out in a staggered arrangement at intervals d1, which are relatively wide in the stretchable direction, while the contraction force of the elastic sheet 30 directly acts on each joining portion 40 to firmly maintain the arrangement with intervals of the joining portions 40 on the positions of the joining holes 31 in the elastic sheet 30, which results in hardly lowered flexibility. Further, the ridges 25f generally extend in the direction perpendicular to the stretchable direction, and each joining portion 40 is concealed between the ridges 25f to be unnoticeable. In this way, the elastic sheet stretchable structure 20X is provided with the appearance still closer to fabric while lowering of flexibility is suppressed.

While the joining portions 40 are laid out in a staggered arrangement, the shape of each joining portion 40 may be circular.

The shape of each joining portion 40 is preferably elongate in the direction orthogonal to the stretchable direction. However, with too short or too long the maximum length of the joining portion 40 in the direction orthogonal to the stretchable direction, the uprightness of the ridges 25f may be deteriorated or the flexibility may be lowered. Thus, though these dimensions may suitably be decided, the length 40y of the joining portion 40 in the direction orthogonal to the stretchable direction is preferably 0.4 to 3.2 mm, particularly 0.7 to 1.4 mm.

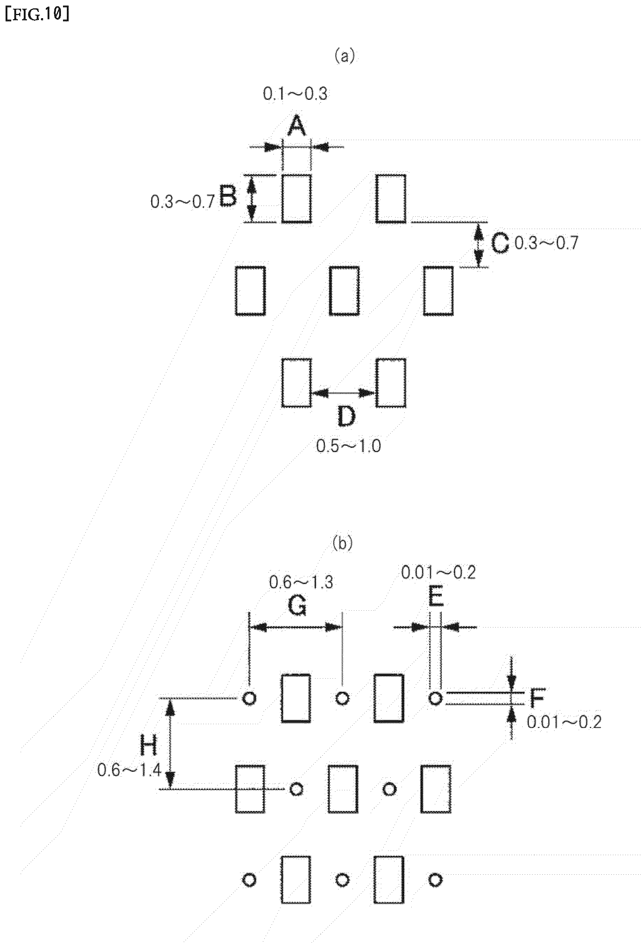

On the other hand, according to Patent Literature 2, in both of the examples shown in Figs. 10(a) and 10(b), joining portions in a stretchable sheet (illustrated as slightly oblong rectangles in the figures) are similarly laid out in a staggered arrangement, and in the example of Fig. 10(b), small round secondary joining portions are arranged among rectangular primary joining portions. The example of Fig. 10(b) is also based on the idea of the staggered arrangement.

The arrangement and dimensions of the joining portions are preferably within the dimensional ranges (in mm) shown in Fig. 10 mainly for appearance, texture, and air permeability.

### (Layout Example 2 of Joining Portions)

According to Layout Example 1 discussed above, since the spaced intervals in the direction orthogonal to the stretchable direction between the joining portions in the elastic sheet 30, indicated by reference sign C in Fig. 10(a), is set at as large as 0.3 mm or more, the stretching stress in the stretchable direction is high and, when such intervals are applied to an underpants-type disposable diaper, not a little wearers may feel excessively (in the width direction) strong constriction.

In this regard, according to Patent Literature 2, the length B of each joining portion indicated in Fig. 10 is preferably 0.3 to 0.7 mm, and the spaced intervals H preferably 0.6 to 1.4 mm.

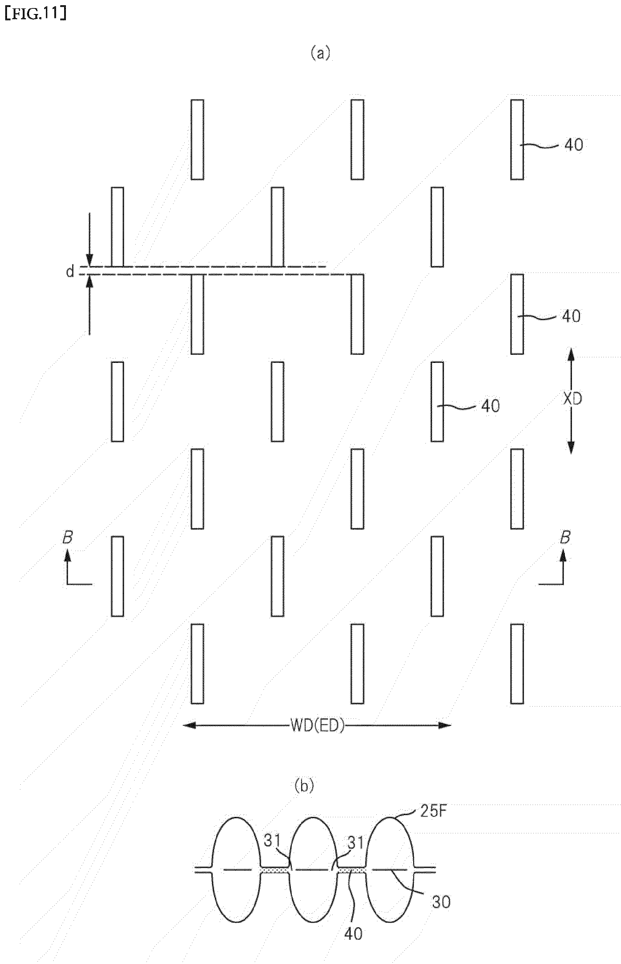

In contrast, as shown in Fig. 11, by setting the spaced intervals d between the joining portions in the elastic sheet 30 at a smaller dimension in the direction XD orthogonal to the stretchable direction ED, the stretching stress in the stretchable direction may be reduced, so that when the spaced intervals are applied to underpants-type disposable diapers, gentle fit on the wearers with weak constriction may be realized.

This is because the joining portions are opened in the width direction to provide the joining holes 31 as shown in Fig. 9 simply upon externally imposing a small stretching force in the width direction (stretchable direction of the elastic sheet 30), whereas when the spaced intervals regions orthogonal to the stretchable direction between the joining portions are stretched in the width direction, the stretching stress of the elastic sheet 30 is assumed to constrict the wearers directly as a contraction force, in the absence of the joining portions.

The embodiment shown in Fig. 11 may realize gentle fit on the wearers, while it provides the advantage of excellent air permeability due to the high area rate of the joining portions and the high area rate of the joining holes in the state of use stretched in the width direction.

### (Layout Example 3 of Joining Portions)

Layout Example 2 discussed above has the advantage of realizing gentle fit on the wearers, but it is sometimes desired to impart a still weaker contraction force. Fig. 25 presents a valid solution to this problem.

In the embodiment shown in Fig. 25, the joining portions 40 are formed in the stretchable regions at intervals in the stretchable direction ED and in the orthogonal direction XD orthogonal thereto,
a group of joining portions 40, 40... in the stretchable regions are in a relation to intersect a stretchable direction line at any position in the orthogonal direction XD as shown in Fig. 25(b), or in a relation not to intersect a stretchable direction line within the spaced intervals of 0.5 mm or less in the orthogonal direction XD orthogonal to the stretchable direction line as shown in Fig. 25(c), and
the group of joining portions are in a relation not to intersect a group in the orthogonal direction XD of slant lines q (i.e., a group of slant lines between the slant lines q, q in Fig. 25(b)) within the predetermined spaced interval in the orthogonal direction XD, each slant line intersecting a stretchable direction line at an angle γ within the range of 45 degrees or less.

The reason why this embodiment does not impose excessive contraction force on the wearers is not necessarily clear, but is assumed to be ascribable to the following phenomenon.

It was assumed that the stretching in the stretchable direction may not occur when the joining portions were in the relation to intersect a stretchable direction line at any position in the orthogonal direction XD as shown in Fig. 25(b), or in the relation not to intersect a stretchable direction line within the spaced intervals d of 0.5 mm or less in the orthogonal direction XD orthogonal to the stretchable direction line.

However, the force in the stretchable direction during expansion of the diaper in the stretchable direction ED upon wearing propagates in a circumventive route as shown in Fig. 25(b) (the propagation route is indicated with reference sign S). This propagation route S is indicated because the elastic sheet 30 stretches and contracts not only in the width direction but also in the orthogonal direction. As a result, stretching in the stretchable direction ED occurs while the joining holes 31, 31 are formed on both sides in the width direction of the joining portion 40.

Generally, when an elastic sheet 30 is stretched during manufacture and the stretching force is then released, the elastic sheet 30 does not return to its initial length, but returns to a length resulting from subtraction of the strain. For example, when an elastic sheet having a natural length of 50 mm is stretched 3.5 times to 175 mm, and upon release of the stretching force, returned to 70 mm, there is a 20 mm strain and the percentage of the strain ε% is (70 - 50) × 100 / 50 = 40%.

Reviewing further based on the above, in the state of a diaper spread in the width direction, stretching in the stretchable direction occurs while the joining holes 31, 31 are formed on both sides in the width direction of each joining portion 40. In other words, on both sides in the width direction of the joining portion 40, deformation of the elastic sheet occurs due to the opening of the joining holes 31, 31. It should be understood that the portion once deformed may have less contracting force.

In this way, when the spreading force of a diaper is released, contraction in the width direction occurs while the opening width (opening length) of the joining holes 31, 31 is being decreased due to the contraction force of the elastic sheet. Here, when the spaced intervals d are large, deformation of the elastic sheet does not occur within the spaced intervals d regions, resulting in a large amount (length) of contraction in the width direction. For example, when the wearer is thin, the diaper is contracted until the joining holes 31, 31 are closed, which may not ensure sufficient air permeability through the openings of the joining holes 31, 31.

On the other hand, when the spaced intervals d are small or zero, deformation of the elastic sheet 30 due to the opening of the joining holes 31, 31 occurs (in a sense, the elastic sheet is damaged) all over or almost all over the orthogonal direction. As a result, when the stretching force in the width direction is released, the opening width (opening length) of the joining holes 31, 31 once opened is short and the rate is low, so that excessive lowering of the air permeability through the joining holes 31, 31 may be avoided.

Further, since the contraction force in the width direction is smaller compared to that with a larger spaced intervals d, excessive compression on the wearer may be avoided.

For the stretching and contraction in the width direction to occur, for example, in the propagation route S as discussed above, it is required that, within the predetermined spaced interval H in the orthogonal direction XD of the orthogonal group of slant lines q, q, which intersect with a stretchable direction line at an angle γ of 45 degrees or less, none of the group of joining portions 40, 40... intersects with any of the slant lines q, q, as shown in Fig. 25(b).

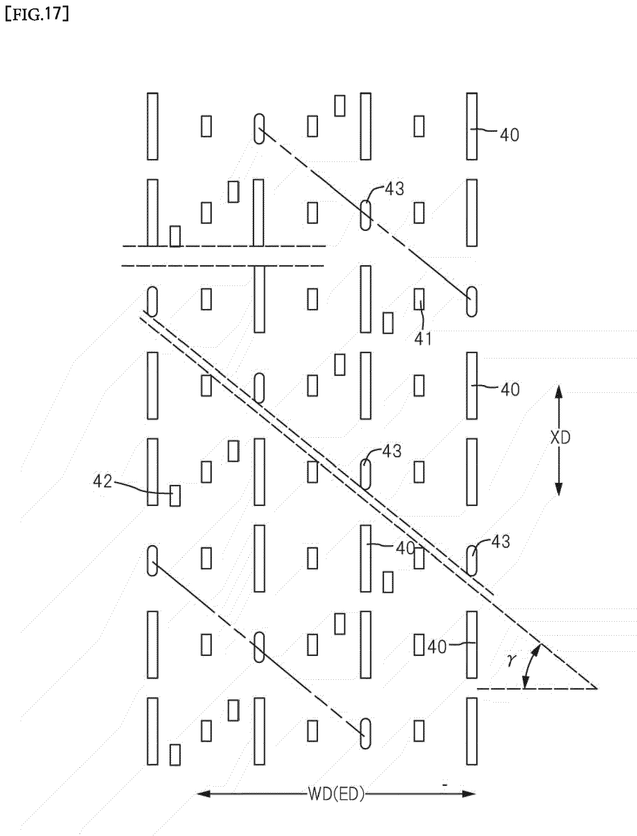

Here, the angle γ of 45 degrees or less with respect to a stretchable direction line is defined as an angle formed between a stretchable direction line and the slant line q, also in the case of slant lines extending from upper left to lower right, for example, as shown in Fig. 17.

The spaced interval H along the orthogonal direction XD is preferably 0.2 mm to 10 mm, more preferably 0.2 to 5.0 mm, particularly preferably 0.6 to 3.0 mm.

The angle γ between a stretchable direction line and the slant line is more preferably 30 degrees or less, particularly preferably 15 degrees or less.

Each joining portion 40 is formed with the width in the stretchable direction of 0.3 to 10.0 mm, preferably 0.5 to 5.0 mm, particularly preferably 0.7 to 3.5 mm.

Each joining portion 40 is formed with the length in terms of the orthogonal direction XD of 0.3 to 7.0 mm, preferably 0.5 to 5.0 mm, particularly preferably 0.7 to 2.5 mm.

Further, lines of first joining portions 40, 40... are arranged with the pitch SO in terms of the stretchable direction ED(WD) of 2.0 to 20.0 mm, preferably 3.0 to 15.0 mm, particularly preferably 4.0 to 10.0 mm.

Various modifications based on Layout Example 3 discussed above will now be described in order hereinbelow.

### (Layout Example 4 of Joining Portions)

Under the conditions of use of a product according to the embodiment shown in Fig. 12(a), in the spaced region between a line of joining portions 40, 40... along the orthogonal direction XD and an adjacent line of joining portions 40, 40... spaced apart therefrom in the stretchable direction ED, ridge 25F extending along the orthogonal direction XD is formed. This ridge 25F has a simple uniform mound-form as shown in Fig. 12(b). That is, the cross section of the ridge 25F is different from the cross section shown in Fig. 9(c) herein and disclosed in Patent Literature 1.

The embodiment shown in Fig. 12 has an elastic sheet stretchable structure wherein an elastic sheet is interposed between an air-permeable first sheet layer and an air-permeable second sheet layer, and the first and second sheet layers are joined at multiple joining portions arranged at intervals through joining holes penetrating the elastic sheet, or via the elastic sheet.

Further, stretchable regions having the elastic sheet stretchable structure are stretchable and contractable in the stretchable direction due to the contraction force of the elastic sheet.

The joining portions include first joining portions 40, 40... as well as second joining portions 41, 41...

The first joining portions 40, 40... are arranged at intervals along the orthogonal direction XD to form lines of first joining portions.

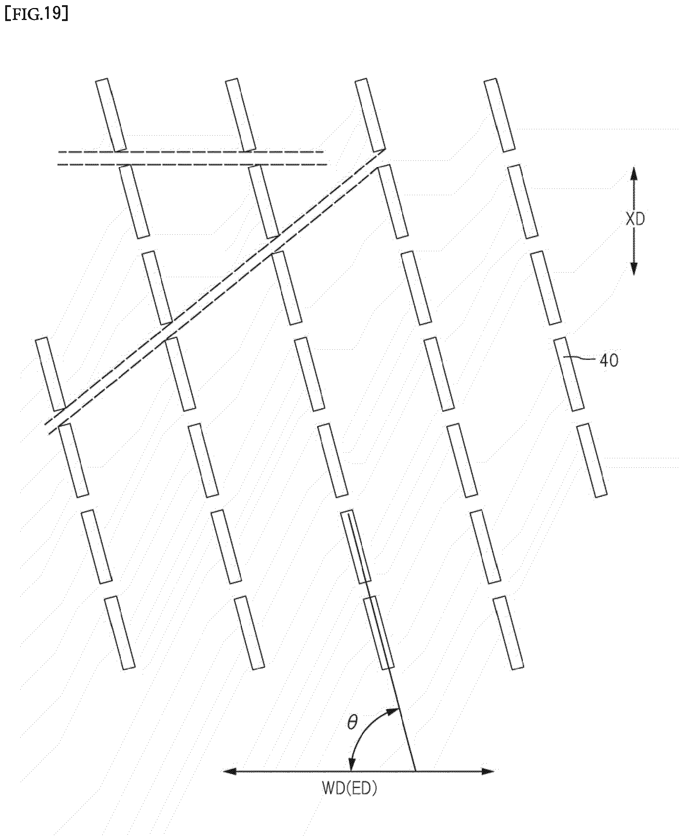

As will be explained later with reference to, for example, Fig. 19, it is preferred that the lines of first joining portions 40, 40... are not along the orthogonal direction XD, but are inclined at an angle θ in the range of 30 to 150 degrees (i.e., excluding 90 degrees), more preferably in the range of 45 to 135 degrees (excluding 90 degrees), with respect to the stretchable direction ED.

The embodiment shown in Fig. 12 is an example wherein the crossing angle θ is 90 degrees, which is not inclined.

The first joining portion 40 is formed with a length in terms of the orthogonal direction XD of 0.3 to 7.0 mm, preferably 0.5 to 5.0 mm, particularly preferably 0.7 to 2.5 mm.

Further, the lines of the first joining portions 40, 40... are arranged with the pitch SO in terms of the stretchable direction ED(WD) of 2.0 to 20.0 mm, preferably 3.0 to 15.0 mm, particularly preferably 4.0 to 10.0 mm.

As a distance in terms of the orthogonal direction XD defined by the relationship between adjacent first joining portions 40, 40... in the lines of first joining portions 40, 40..., the ratio in percentage of (spaced interval d between adjacent first joining portions) / (distance P between a point on a joining portion and a corresponding point on an adjacent first joining portion) is 5 to 60%, preferably 10 to 45%, particularly preferably 20 to 35%.

At an excessively high percentage, upon application of the layout to a product, the stretching stress in the width direction (stretchable direction) tends to be too high to provide proper fitting as a wearing article.

On the other hand, at an excessively low percentage, a possibility of the first joining portions 40, 40 adjacent to each other in the orthogonal direction XD becoming continuous during the manufacturing process may not be eliminated and, ultimately, excessive equipment burden may be imposed on anvils and heating horns for forming the joining portions, to thereby disturb stable operation.

It is preferred that in the lines of the second joining portions 41, 41, joining portions having the length L of the first joining portions 40 or longer are not formed.

This embodiment typically has the following advantages or features:
(1) Since the percentage discussed above is low, a stretchable sheet member may be obtained having a low stretching stress in the stretchable direction and providing flexible stretching, and when applied to absorbent articles, provides excellent feeling of wearing.
   In addition, the opening ratio is high, which leads to high air permeability.
(2) Since not only the lines of first joining portions 40, 40... but also the lines of second joining portions 41, 41... are formed, pleat R between the lines may be formed between a line of first joining portions 40, 40...and a line of second joining portions 41, 41... In the previous embodiment shown in Fig. 11, in terms of design of the overall stretchable regions of a product, the ridges 25F elongate in the orthogonal direction XD are formed in the stretchable direction ED(WD) in a uniformly repetitive fashion, whereas in the present embodiment, formation of the pleats R between the lines may improve the design.
(3) The second joining portions 41 are smaller in area than the first joining portions 40, which gives patterned appearance.
(4) As the pleat R between the lines are formed between a line of first joining portions 40, 40... and a line of second joining portions 41, 41..., two pleats R between the lines may be formed between the lines of first joining portions 40, 40.... Here, in a line of second joining portions 41, 41..., the distance between the second joining portions 41, 41 is longer, which means that the pleats may be formed without excessive equipment burden on anvils and heating horns. As a result, compared to the case where the pleats between the lines are formed by only the lines of first joining portions 40, 40... as shown in Fig. 11, a larger number of thinner pleats may be formed per unit area without equipment burden.
   In this way, the area of contact with the skin of the wearer may be reduced, which improves comfort and softness.

### (Layout Example 5 of Joining Portions)

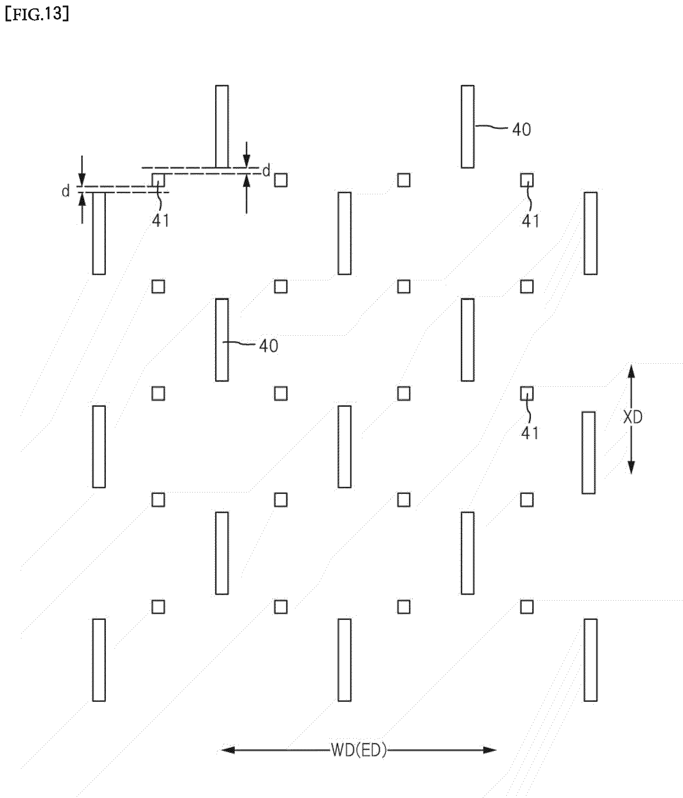

As shown in Fig. 13, each of a group of second joining portions 41, 41... may be disposed between the first joining portions 40, 40... in terms of the orthogonal direction XD. In this case, though the length L of each first joining portions 40 is short, the presence of the second joining portions 41 may reduce the stretching stress.

### (Layout Example 6 of Joining Portions)

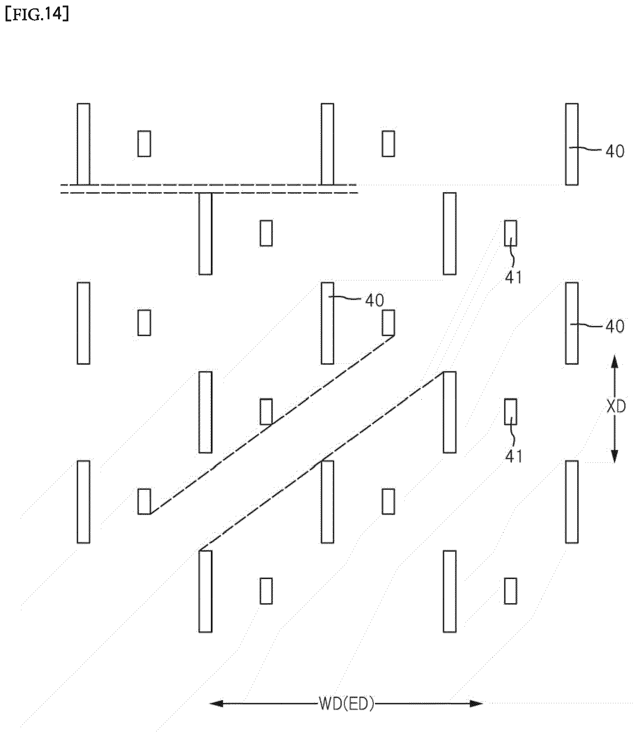

As shown in Fig. 14, the second joining portions 41 are not necessarily juxtaposed to the first joining portions 40 on a one-to-one basis and, for example, two of the second joining portions 41, 41 may be juxtaposed to one of the first joining portions 40, 40.

### (Layout Example 7 of Joining Portions)

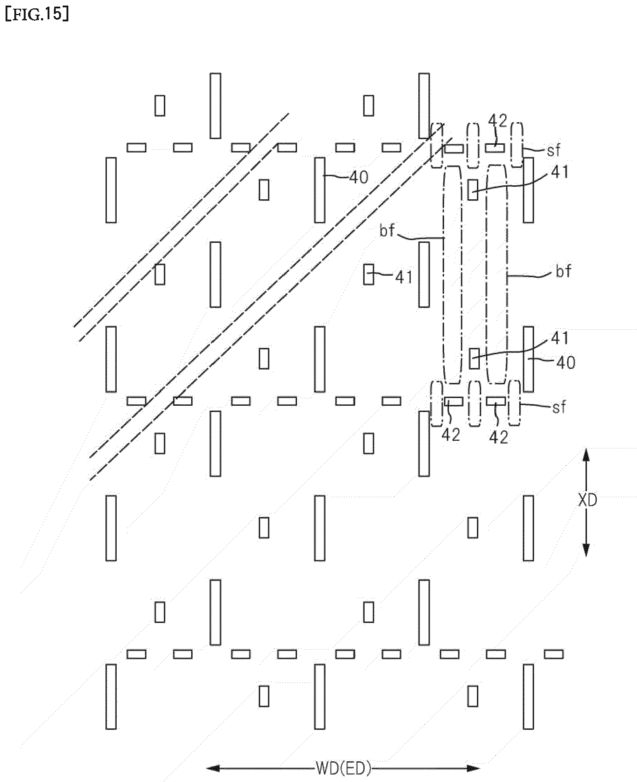

As shown in Fig. 15, a line of third joining portions 42, 42... at longer spaced intervals in the orthogonal direction XD may be formed between a line of first joining portions 40, 40... and a line of second joining portions 41, 41.... By forming the third joining portions 42, the pleat R between the lines in the first embodiment may be divided in the orthogonal direction XD to form large pleats bf . Further, small pleats sf may be formed between the third joining portions 42 and a line of first joining portions 40, 40... The group of pleats formed by dividing the pleats R between the lines imparts to the stretchable member a low bending rigidity (easier to bend) and good ability to follow the body movement.

### (Layout Example 8 of Joining Portions)

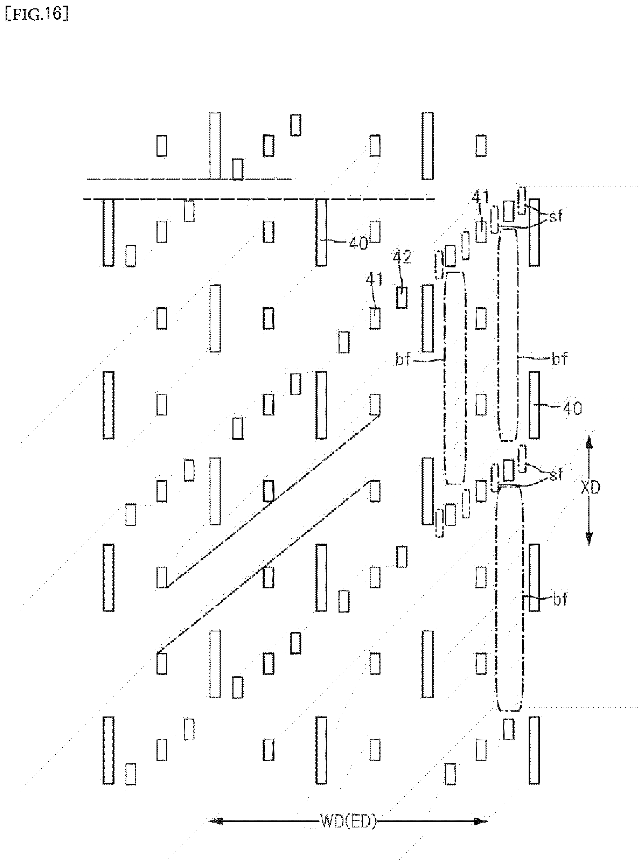

As shown in Fig. 16, by forming the third joining portions 42 in oblique arrangements with the second joining portions 41, a group of large pleats bf arranged obliquely may be formed to improve the design.

### (Layout Example 9 of Joining Portions)

As shown in Fig. 17, fourth joining portions 43 may be inserted and arranged in a line of first joining portions 40, 40.... Here, the group of fourth joining portions 43, 43... may be arranged along the stretchable direction ED as well as obliquely as shown in the figure. In this case, the area of the fourth joining portions 43 is preferably 5% or more and 50% or less of the area of the first joining portions 40.

### (Layout Example 10 of Joining Portions)

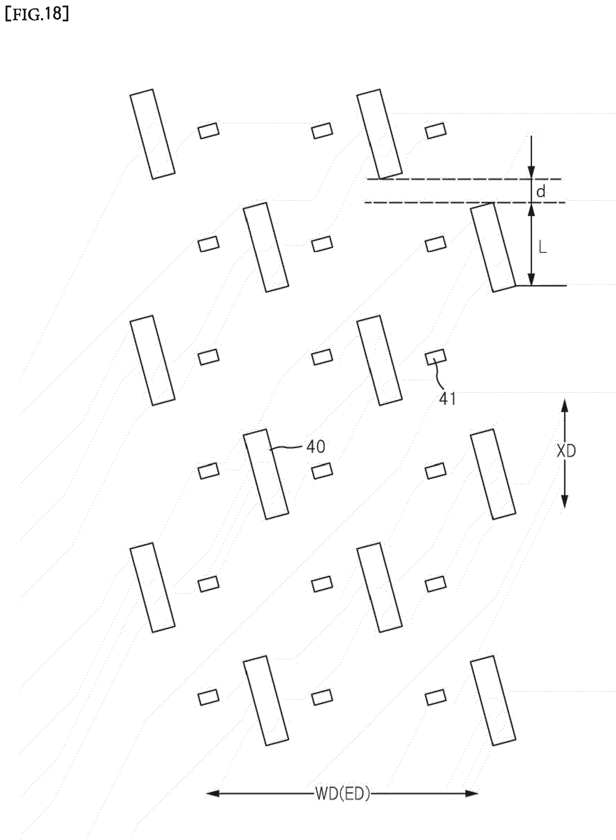

As shown in Fig. 18, each first joining portion 40 per se may be slanted. Each second joining portion 41 per se may also be slanted. Since the length of a joining portion is in terms of the orthogonal direction XD, the length L of the first joining portion 40 is the length between the center of one side and the center of the other side in the orthogonal direction XD, as shown in Fig. 18. As to the spaced interval, the distance between the center of one side and the center of the facing side in the orthogonal direction XD is taken as the spaced interval d.

### (Layout Example 11 of Joining Portions)

Fig. 19 shows an embodiment wherein each first joining portion 40 is slanted, and each line of the joining portions is not along the orthogonal direction XD but is slanted at an angle θ of 30 to 150 degrees, preferably 45 to 135 degrees, with respect to the stretchable direction ED. The crossing angle θ is particularly preferably 60 to 120 degrees. In this regard, however, the angle range expressing the slant obviously excludes 90 degrees.

The advantage of the lines of the joining portions being slanted and crossing the stretchable direction ED without extending along the orthogonal direction XD may be apparent from the comparison with Layout Example 10 illustrated in Fig. 18. That is, in the embodiment shown in Fig. 19, for example, the spaced intervals between the first joining portions 40, 40 along the orthogonal direction XD line are much larger than those in the Layout Example 10 illustrated in Fig. 18, which results in the advantage.

For example, the joining between the first sheet layer 20A and the second sheet layer 20B in the joining portions 40 may preferably be performed by joining means through material welding, such as heat sealing or ultrasonic sealing.

In continuous production, ultrasonic seal melting is performed between an anvil roll and an ultrasonic horn. In order to avoid energy loss, it is important that the ultrasonic horn is in close contact with the sheet all along the axial direction of the anvil roll. For forming a pattern requiring a large ratio of convex portions on the anvil roll along the generatrix in line contact, like the lines of joining portions 40, 40 in Fig. 12, a large ultrasonic output is required, and acting an excessive closely contacting force along the generatrix in line contact for this purpose imposes a large equipment burden.

In contrast, according to this embodiment (in the oblique arrangement in general), the ratio of the joining portions located on a line in the orthogonal direction XD is small, so that the linear pressure is stable, which results in smaller equipment burden and realizes stable operation. Further according to this embodiment, as each first joining portion 40 (as well as each second joining portion 41) is slanted, formation of ridges and pleats with excellent design may also be advantageously realized.

### (Non-stretchable Region)

The regions of the outer member 20 having the elastic sheet stretchable structure 20X may be provided with non-stretchable regions 70 in addition to the stretchable regions 80, as shown in Fig. 7. A non-stretchable region 70 means that the elongation at elastic limit in the stretchable direction of the region is 120% or less. The elongation at elastic limit of the non-stretchable regions 70 is preferably 110% or less, more preferably 100%. The arrangement of the stretchable regions 80 and the non-stretchable regions 70 may suitably be decided. In the case of the outer member 20 of an underpants-type disposable diaper as shown in the figure, since the regions overlapping the absorbent body 13 are not required to be stretchable, it is preferred to make a part or all of the regions overlapping the absorbent body 13 (preferably including most of the inner-outer fixed region 10B) the non-stretchable regions 70, as in the illustrated embodiment. It is indisputable that the non-stretchable regions 70 may be arranged from the region overlapping the absorbent body 13 toward the region not overlapping the absorbent body 13 located in the width direction WD or the front-back direction LD to the overlapping region, or may be arranged only in the regions not overlapping the absorbent body 13.

In the non-elastic regions 70, the respective shape and arrangement of the joining portion 40 and the respective shape and arrangement of the joining hole 31 in the elastic sheet 30 may suitably be decided.

Further in the non-elastic regions, the area rate of the joining portions 40 and the area of each joining portion 40 may suitably be decided, and may usually be preferred to fall within the following ranges for keeping the non-stretchable regions 70 from becoming hard owing to the small area of each joining portion 40 and the low area rate of the joining portions 40:

Area of each joining portion 40: 0.10 to 0.75 mm² (particularly 0.10 to 0.35 mm²)
Area rate of joining portions 40: 4 to 13% (particularly 5 to 10%)

The non-stretchable regions 70 may be formed by densely arranging the joining portions 40, or the like means, so that the ridges are not formed by contraction of the first and the second sheet layers due to the contraction force of the elastic sheet 30. Specific examples of the means for forming the non-stretchable regions 70 may be found in, for example, JP-5980355-B, JP-5918877-B, JP-5980367-B, or JP-6049228-B.

In particular, it is preferred that, due to the presence of the joining holes 31, the non-stretchable regions 70 do not have any portion in which the elastic sheet 30 is linearly continuous along the width direction WD, irrespective of the continuous extension of the elastic sheet 30 in the width direction WD. In this case, even if the overall elastic sheet stretchable structure 20X including both the stretchable regions 80 and the non-stretchable regions 70 is formed by, with the elastic sheet 30 being stretched in the width direction WD, joining the first sheet layer 20A and the second sheet layer 20B through the joining holes 31 in the elastic sheet 30 at intervals in the width direction WD and in the front-back direction LD orthogonal thereto, to thereby form multiple joining portions 40, the elastic sheet 30 is not linearly continuous along the width direction WD in the non-stretchable regions 70, so that the contraction force of the elastic sheet 30 hardly acts on the first sheet layer 20A and the second sheet layer 20B, which almost disperses the stretchability and makes the elongation at elastic limit to be nearly 100%.

In such non-stretchable regions 70, as the first sheet layer 20A and the second sheet layer 20B are joined at multiple joining portions 40 arranged at intervals to interrupt continuity of the elastic portions 40, deterioration of flexibility is avoided.

### (Joint Structure of Joining Portion)

Joining of the first sheet layer 20A and the second sheet layer 20B at the joining portions 40, when made through the joining holes 31 in the elastic sheet 30, is preferably performed such that the first sheet layer 20A and the second sheet layer 20B are not joined to the elastic sheet 30 except for at least the first sheet layer 20A and the second sheet layer 20B at the joining portions 40.

The means for joining the first sheet layer 20A and the second sheet layer 20B at the joining portion 40 are not particularly limited. For example, the joining of the first sheet layer 20A and the second sheet layer 20B at the joining portions 40 may be made by means of a hot melt adhesive or joining means employing material welding, such as heat sealing or ultrasonic sealing.

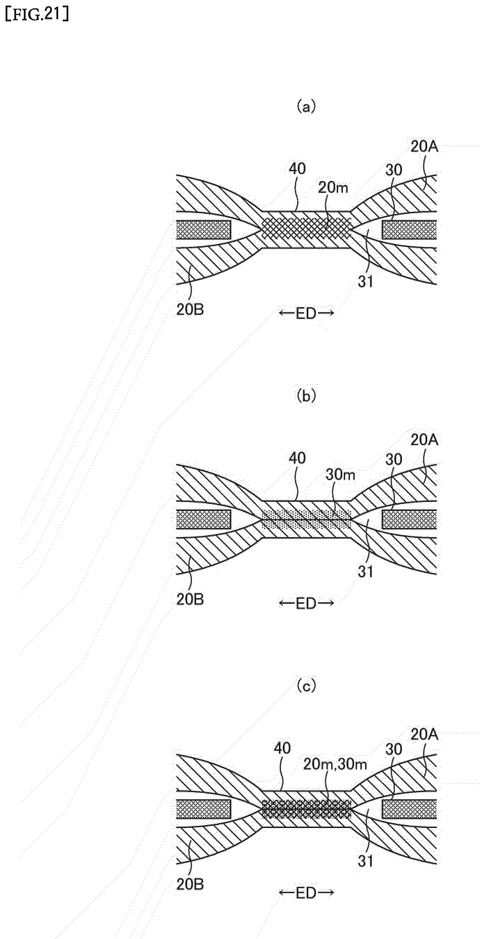

When the first sheet layer 20A and the second sheet layer 20B are joined at the joining portions 40 through the joining holes 31 in the elastic sheet 30, the structure of the joining portions 40 formed by material welding may be any of first weld mode wherein the first sheet layer 20A and the second sheet layer 20B are joined only by solidified molten products 20m of most or part of at least one of the first sheet layer 20A and the second sheet layer 20B at the joining portions 40 (see Fig. 21(a)), second weld mode wherein the first sheet layer 20A and the second sheet layer 20B are joined only by solidified molten products 30m of all, most, or part of the elastic sheet 30 at the joining portions 40 (see Fig. 21(b)), and third weld mode, which is the combination of these structures (see Fig. 21(c)). Among these, the second and the third structures are preferred.

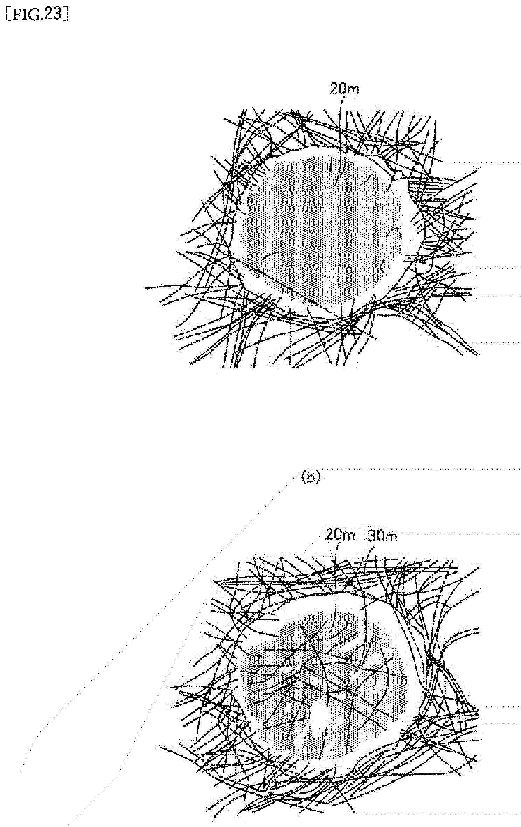

Particularly preferred is a structure wherein the first sheet layer 20A and the second sheet layer 20B are joined by the solidified molten products 20m of part of the first sheet layer 20A and the second sheet layer 20B as well as the solidified molten products 30m of all or most of the elastic sheet 30 at the joining portions 40. Incidentally, in the third weld mode shown in Fig. 23(b), among the solidified molten products 20m of the fibers of the first sheet layer 20A and the second sheet layer 20B shown in black, the solidified molten products 30m of the elastic sheet 30 shown in white may be observed, whereas in the first weld mode shown in Fig. 23(a), among the solidified molten products 20m of the fibers of the first sheet layer 20A and the second sheet layer 20B, no solidified molten product of the elastic sheet 30 may be observed.

As in the first weld mode or the third weld mode, when the first sheet layer 20A and the second sheet layer 20B are joined by means of the solidified molten products 20m of most or part of at least one of the first sheet layer 20A and the second sheet layer 20B as an adhesive, it is preferred that part of the first sheet layer 20A and the second sheet layer 20B is not molten, as the joining portions 40 are not hardened.

When the first sheet layer 20A and the second sheet layer 20B are of nonwoven fabric, the joint structure wherein part of the first sheet layer 20A and the second sheet layer 20B are not molten, includes a structure wherein the cores (including not only the cores of composite fibers, but also the central portions of single component fibers) of all the fibers at the joining portions 40 remain while the portions around the cores (including not only the sheathes of composite fibers, but also the surficial portions of single component fibers) are molten, and a structure wherein part of the fibers are not molten at all, but the remaining fibers are molten in their entirety or the cores remain while the portions around the cores are molten.

As in the second weld mode and the third weld mode, use of the solidified molten products 30m of the elastic sheet 30 as an adhesive for joining the first sheet layer 20A and the second sheet layer 20B results in high peel strength. The second weld mode may be formed by interposing the elastic sheet 30 between the first sheet layer 20A and the second sheet layer 20B wherein the melting point of at least one of the first sheet layer 20A and the second sheet layer 20B is higher than the melting point of the elastic sheet 30 and the heating temperature for forming the joining portions 40, and heating under pressure the portions to be the joining portions 40 under, to thereby melt only the elastic sheet 30.

On the other hand, the third weld mode may be formed by interposing the elastic sheet 30 between the first sheet layer 20A and the second sheet layer 20B wherein the melting point of at least one of the first sheet layer 20A and the second sheet layer 20B is higher than the melting point of the elastic sheet 30 and heating under pressure the portions to be the joining portions 40, to thereby melt at least one of the first sheet layer 20A and the second sheet layer 20B as well as the elastic sheet 30.

In view of the above, the melting point of the elastic sheet 30 is preferably about 80 to 145°C, the melting points of the first sheet layer 20A and the second sheet layer 20B are preferably about 85 to 190°C, particularly preferably 150 to 190°C, and the difference between the melting point of the elastic sheet 30 and the melting points of the first sheet layer 20A and the second sheet layer 20B is preferably about 60 to 90°C. The heating temperature is preferably about 100 to 150°C.

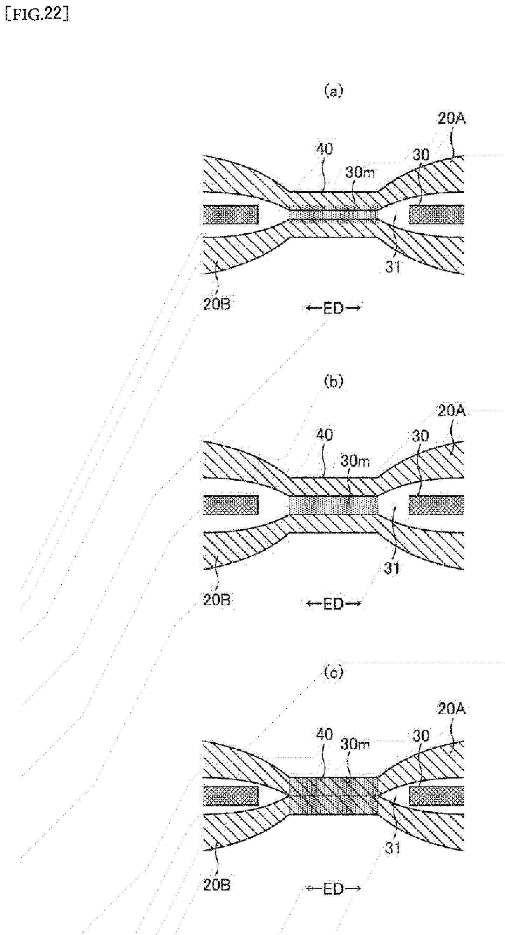

In the second weld mode and the third weld mode, with the first sheet layer 20A and the second sheet layer 20B being of nonwoven fabric, the solidified molten product 30m of the elastic sheet 30 may permeate the fibers all over the thicknesses of the first sheet layer 20A and the second sheet layer 20B at the joining portions 40 as shown in Fig. 22(c). However, higher flexibility of the joining portions 40 may be achieved in the structure in which the permeation is halfway through the thicknesses as shown in Fig. 22(a), or the structure in which the permeation hardly occurs into the fibers of the first sheet layer 20A and the second sheet layer 20B as shown in Fig. 22(b).

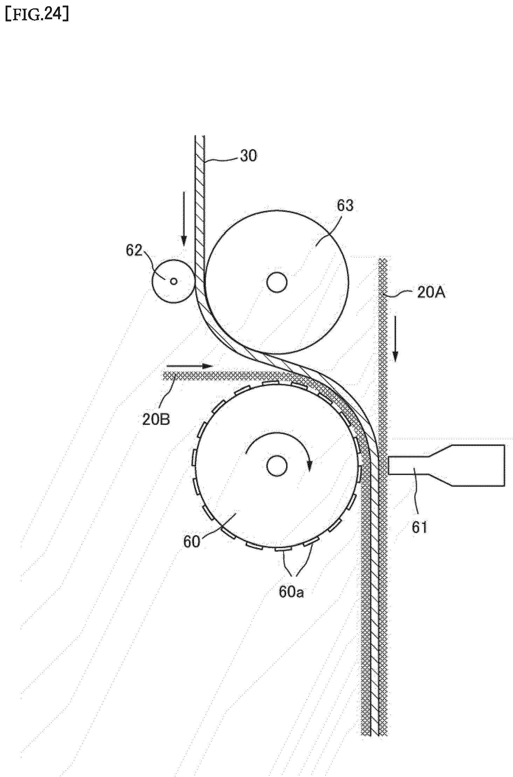

Fig. 24 illustrates an example of ultrasonic sealing apparatus preferred for the second weld mode and the third weld mode. In this ultrasonic sealing apparatus, for forming the joining portions 40, the first sheet layer 20A, the elastic sheet 30, and the second sheet layer 20B are fed between the ultrasonic horn 61 and the anvil roll 60 having protrusions 60a on its periphery corresponding to the pattern of the joining portions 40. Here, for example, by setting the feeding transfer rate of the feeding driving roller 63 and the nip roller 62 for feeding the elastic sheet 30 on the upstream side to a slower rate compared to the transfer rate at and after the anvil roll 60 and the ultrasonic horn 61, the elastic sheet 30 is stretched in the MD direction (machine direction, flow direction) to a predetermined stretch rate in the pathway from the nipping position between the feeding driving roller 63 and the nip roller 62 to the sealing position between the anvil roll 60 and the ultrasonic horn 61. The stretch rate of the elastic sheet 30 may be set by selecting the velocity difference between the anvil roll 60 and the feeding driving roller 63, and may be, for example, about 300% to 500%.

The first sheet layer 20A, the elastic sheet 30, and the second sheet layer 20B fed between the anvil roll 60 and the ultrasonic horn 61 are, in the state laid one on top of another in this order, pressed between the protrusions 60a and the ultrasonic horn 61 while heated with the ultrasonic vibration energy from the ultrasonic horn 61, to melt only the elastic sheet 30 or the elastic sheet 30 and at least one of the first sheet layer 20A and the second sheet layer 20B, to thereby form the joining holes 31 in the elastic sheet 30, while the first sheet layer 20A and the second sheet layer 20B are joined through the joining holes 31. Accordingly, in this case, the area rate of the joining portions 40 may be selected by the selection of the size, shape, spaced intervals, arrangement pattern in the roll length direction and the roll circumferential direction, of the protrusions 60a on the anvil roll 60.

The reason for the joining holes 31 to be formed is not necessarily clear, but it is assumed that the portions of the elastic sheet 30 corresponding to the protrusions 60a on the anvil roll 60 are welded and removed from the around to form open holes. In this regard, the portion of the elastic sheet 30 between the joining holes 31 adjacent to each other in the starching-contracting direction ED is, as shown in Figs. 9(a) and 11(a), is disconnected from the portions on its both sides in the stretchable direction by the joining holes 31 to lose support on its both sides in the contracting direction and, accordingly, contracts more in the area closer to the center in the direction LD orthogonal to the stretchable direction ED until it is balanced on the center in the stretchable direction as far as the continuity in the direction orthogonal to the contracting direction can be maintained, so that the joining holes 31 are expanded in the stretchable direction ED.

The constituting materials of the first sheet layer 20A and the second sheet layer 20B are not particularly limited as long as the materials are in a sheet form, and nonwoven fabric is preferably used for air permeability and flexibility. The nonwoven fabric, when adopted, preferably has a basis weight of about 10 to 25 g/m².

Further, part or all of the first sheet layer 20A and the second sheet layer 20B may be a pair of facing layers formed by folding a sheet of material. For example, as shown in the drawings, in the waist end portions 23, the constituting material located externally may be referred to as the second sheet layer 20B, the folded portion 20C formed by folding the constituting material onto the internal surface side at each waist opening edge may be referred to as the first sheet layer 20A, and the elastic sheet 30 may be interposed therebetween, whereas in the remaining portions, the constituting material located internally may be referred to as the first sheet layer 20A, the constituting material located externally may be referred to as the second sheet layer 20B, and the elastic sheet 30 may be interposed therebetween. It is indisputable that the constituting material of the first sheet layer 20A and the constituting material of the second sheet layer 20B may be separately provided all over the front-back direction LD, and the elastic sheet 30 may be interposed between the constituting material of the first sheet layer 20A and the constituting material of the second sheet layer 20B without folding the constituting materials.

The elastic sheet 30 is not particularly limited, and may be of an elastic film or a stretchable nonwoven fabric, as long as it is a sheet of a thermoplastic resin which elastically stretches and contracts by itself. Further, the elastic sheet 30 may be non-porous, or may be provided with multiple pores or slits for air permeability. In particular, the elastic sheet 30 preferably has a tensile strength in the width direction WD (stretchable direction ED, MD direction) of 8 to 25 N/35 mm, a tensile strength in the front-back direction LD (direction XD orthogonal to the stretchable direction, CD direction) of 5 to 20 N/35 mm, a tensile elongation in the width direction WD of 450 to 1050%, and a tensile elongation in the front-back direction LD of 450 to 1400%. The thickness of the elastic sheet 30 is not particularly limited, and is preferably about 20 to 40 µm. Moreover, an elastic film is provided with an elastic nonwoven fabric on either or both of its surfaces, which may be interposed as the elastic sheet 30 between the first sheet layer 20A and the second sheet layer 20B.

### (Coloring)

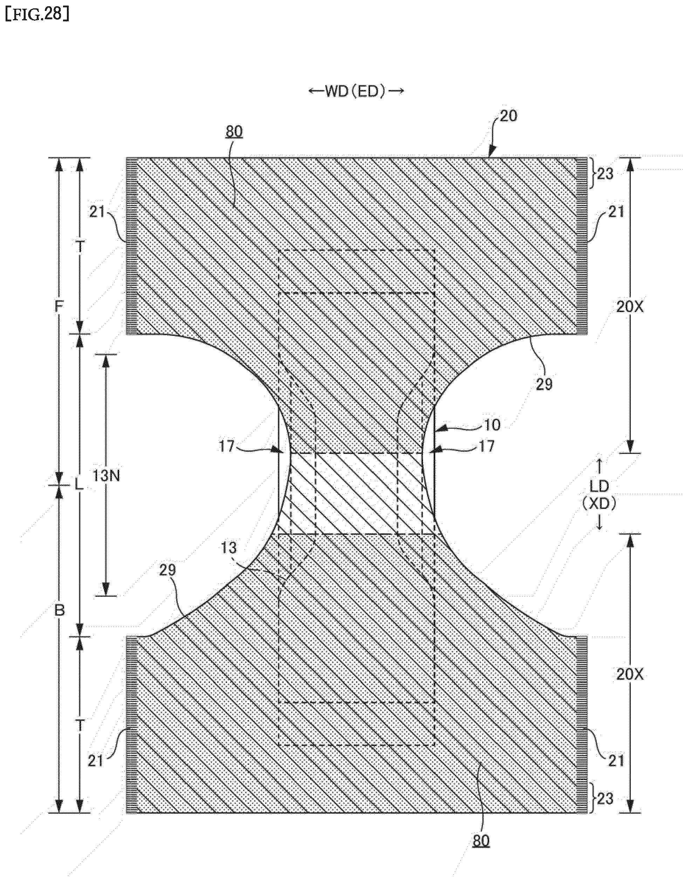

In order to make the present underpants-type disposable diaper a colored product, the second sheet layer 20B having portions exposed to the product external surface may be of a colored nonwoven fabric. In the embodiments shown in the drawings, in the regions having the elastic sheet stretchable structure 20X, the elastic sheet 30 is the base sheet internally adjacent to the colored nonwoven fabric, whereas in the regions without the elastic sheet 30, the first sheet layer 20A is the base sheet internally adjacent to the colored nonwoven fabric. Note that the exposed portions of the second sheet layer 20B in the illustrated embodiments are shown hatched from upper left to lower right in Fig. 28, and the regions having the second sheet layer 20B and the elastic sheet 30 overlapping are shown dotted in Fig. 28.

In the embodiments shown in the drawings, most of the product external surface is covered with the second sheet layer 20B. However, as discussed above, in an underpants-type disposable diaper of a type in which the separate front body F and back body B form the outer member 20 and are arranged in the crotch portion spaced apart from each other in the front-back direction LD of the diaper, the outer member 20 is not present between the front side outer member 20 and the back side outer member 20, so that an outer nonwoven fabric (corresponding to cover sheet 53 disclosed in Patent Literature 1, not shown) exposed to the portion without the outer member 20 is preferably a colored nonwoven fabric. Here, the member internally adjacent to the colored nonwoven fabric is usually the liquid impervious sheet 12. Further, in a so-called tape-type disposable diaper, generally the back surface side of the absorbent body 13 is covered with the liquid impervious sheet 12, such as a waterproof film, and the back surface side of the liquid impervious sheet 12 is in turn covered with an outer nonwoven fabric. Accordingly, in this case, a colored nonwoven fabric is preferably used as the outer nonwoven fabric (not shown). In this way, the colored nonwoven fabric and the base sheet are not particularly limited and, for example, the top sheet 11 may be of a colored nonwoven fabric, and the gather sheet 95 may be of a colored nonwoven fabric.

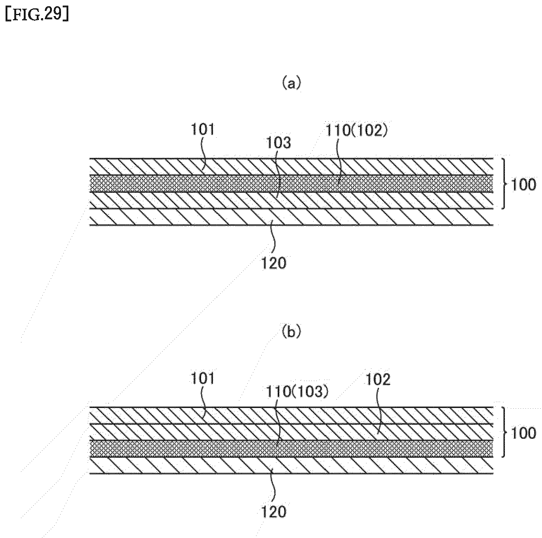

As shown in Fig. 29, the colored nonwoven fabric 100 is a composite nonwoven fabric composed of a plurality of layers laid one on top of another, and has, in addition to outermost layer 101, colored layer 110 colored in a color of the first lightness other than white, and the outermost layer 101 preferably has a color of the second lightness higher than the first lightness. Here, in visual observation of the colored nonwoven fabric 100 from outside, the colored layer 100 is observed through the outermost layer 101, and thus the color of the colored layer 110 is observed as being thinned (lightened). In addition, the outermost layer 101 naturally has irregular formation, which causes unevenness in the thinning degree of the visually observed color of the colored layer 110. As a result, not only the visual color of the colored layer 110 is thinned, but also the color unevenness thereof is made finer, so that the color unevenness of the colored layer 110 is unexpectedly obscured even when the external surface of the base sheet is in the color of the third lightness higher than the first lightness (i.e., the color unevenness of the colored nonwoven fabric 100 is apparent). Consequently, the visual color unevenness of the colored nonwoven fabric 100 may be obscured irrespective of whether the base sheet 120 is colored or not.

In particular, when the colored nonwoven fabric 100 is adopted as the second sheet layer 20B in the elastic sheet stretchable structure 20X as of the present disposable wearable article, the elastic sheet 30 is the base sheet 120 internally adjacent to the colored nonwoven fabric 100 in the regions having the elastic sheet stretchable structure 20X. In this case, the joining holes 31 in the elastic sheet 30 are also observable through the colored nonwoven fabric 100, and may thus adversely affect the appearance. For example, some people may see the joining holes 31 of different sizes as visually non-uniform pattern, like color unevenness. Thus, when the elastic sheet stretchable structure 20X has the above-mentioned color unevenness preventive structure, not only the color unevenness of the colored nonwoven fabric 100 but also the joining holes 31 may preferably be obscured.

The colored nonwoven fabric 100 may be colored all over the direction orthogonal to the thickness direction, or only in a partial region, such as the exposed portions or a region including the portions overlapping the base sheet 120, as long as the colored nonwoven fabric 100 has a colored portion colored in the same color. When the colored portion is provided in the regions having the elastic sheet stretchable structure 20X, the colored portion may either be in the stretchable regions 80 or the non-stretchable regions 70. However, the color unevenness due to the irregular formation of the nonwoven fabric becomes particularly apparent from the area of about 25 cm² of the colored portion, and adaptation of the color unevenness preventive structure discussed above to a diaper having a colored portion of the area larger than this is of technical significance. Further, though not shown, the colored nonwoven fabric 100 may be provided with the colored portions of the same color at a plurality of locations at intervals, or the colored portions of different colors at a plurality of locations at intervals or adjacent to each other. For example, the intermediate portion L and the both end portions in the width direction WD may be colored in different colors, or the waist end portions 23 regions and the remaining regions may be colored in different colors.

The method for manufacturing the colored nonwoven fabric 100 is not particularly limited, and coloring of a single material into a plurality of colors may be carried out by means of printing or piece-dyeing, whereas coloring of the entire material into a single color may also be carried out by means of printing or piece-dyeing, but preferably by means of mixing of dyes or pigments in the stock material (for example, nonwoven fabric is produced from spun-dyed fibers obtained by coloring the spinning solution with dyes or pigments before spinning). The latter means is preferred since whether to color or not may easily be selected for each layer of the composite nonwoven fabric by selecting the raw material with or without coloring for each layer, but the resulting colored nonwoven fabric 100 is naturally colored in the same color all over. It is also possible to take measures to print only on the internal surface of the composite nonwoven fabric so that the coloring component does not reach the outermost layer 101, by which colored nonwoven fabric 100 having partially colored portions or colored nonwoven fabric 100 having a plurality of colored potions in different colors may be obtained.

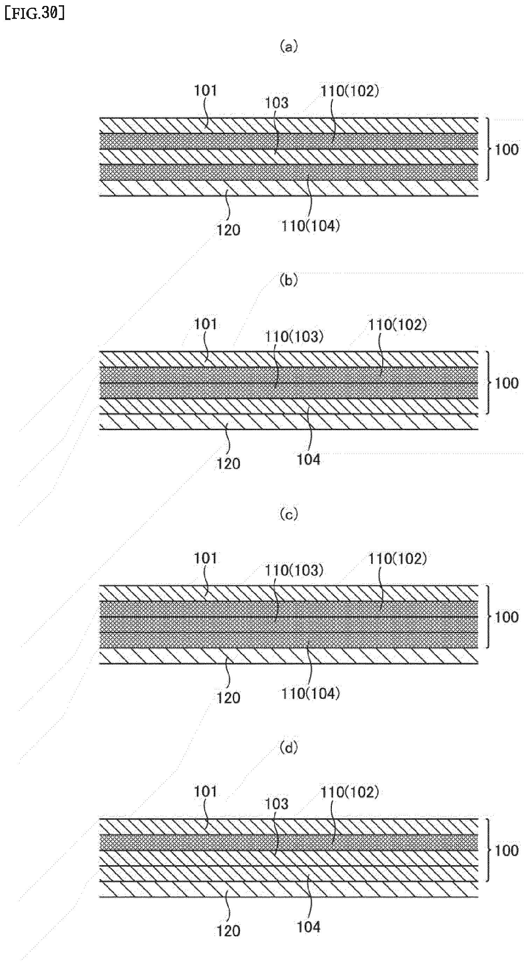

The layered structure of the colored nonwoven fabric 100 is not particularly limited as long as the structure includes, in addition to the outermost layer 101, the colored layer 110 colored in a color of the first lightness other than white, and the outermost layer 101 has a color of the second lightness higher than the first lightness. For example, when the colored nonwoven fabric 100 has a triple-layered structure, the second layer 102 may be the colored layer 110 while the outermost layer 101 and the innermost layer 103 may be non-colored layers of the second lightness as shown in Fig. 29(a), or the innermost layer 103 may be the colored layer 110 while the outermost layer 101 and the second layer 102 may be non-colored layers of the second lightness as shown in Fig. 29(b). When the colored nonwoven fabric 100 has a quadruple-layered structure, the second layer 102 and the fourth layer 104 from the external side may be the colored layers 110 while the first layer (the outermost layer 101) and the third layer 103 may be the non-colored layers of the second lightness as shown in Fig. 30(a), or the second layer 102 and the third layer 103 from the external side may be the colored layers 110 while the first layer (the outermost layer 101) and the fourth layer 104 may be the non-colored layers of the second lightness as shown in Fig. 30(b). Further, except for the outermost layer, all the layers 102 to 104 may be the colored layers 110 as shown in Fig. 30(c), or in a structure having four or more layers, only one layer may be the colored layer 110 as shown in Fig. 30(d). That is, the number of the colored layers 110 and the non-colored layers is not limited.

The color of the first lightness, the color of the second lightness, and the color of the third lightness are not particularly limited, and it is preferred, for example, that the color of the first lightness has a CIELAB L* value of 20 to 90, and the absolute value of at least one of a* and b* values is 0 to 40. Specifically, the colored layer 110 may be colored in a color of a relatively low lightness, such as beige, gray, pink, blue, purple, or yellow.

The color of the second lightness preferably has a CIELAB L* value of 60 to 100. It is preferred that the color of the second lightness is or close to white. Specifically, the absolute value of at least one of a* and b* values of the color of the second lightness is 0 to 5. The color in the non-colored state (i.e., the color of the material per se) of the outermost layer 101 of the colored nonwoven fabric 100 is preferably within the ranges mentioned above. Irrespective of the above, the color of the outermost layer 101 of the colored nonwoven fabric 100 and the color of the base sheet 120, in the non-colored state, are not within the above-mentioned ranges, the color of the outermost layer 101 of the colored nonwoven fabric 100 may be adjusted to the lightness within the above-mentioned ranges by, for example, addition of a white pigment.

When the color of the third lightness has a CIELAB L* value of 60 to 100, color unevenness of the colored nonwoven fabric 100 tends to be apparent. When the color of the third lightness is or close to white, for example, when the absolute value of at least one of a* and b* values of the color of the third lightness is 0 to 5, color unevenness of the colored nonwoven fabric 100 is particularly apparent. In such a case, in particular, having the above-mentioned color unevenness preventive structure is of a particular technical significance. It is indisputable that, when a plurality of base sheets 120 are present as in the present embodiment, it is technically significant even if only a part of the sheets has the third lightness, and the technical significance is even more when all of the sheets have the third lightness.

The color of the second lightness and the color of the third lightness may be different colors or of the same type of color. In particular, when the color of the outermost layer 101 of the colored nonwoven fabric 100 is close to the color of the base sheet 120, higher color unevenness preventive effect may be achieved. Accordingly, the color difference **ΔE** between the colors of the second and the third lightness is preferably 30 or less.

For soft texture, preferably the colored nonwoven fabric 100 is a composite nonwoven fabric of 2 to 4 layers having a fineness of 1.5 to 5.0 dtex and a basis weight of 10 to 20 g/m², but in this case, formation of the nonwoven fabric tends to appear as color unevenness. Irrespective of this, with the above-mentioned color unevenness preventive structure, color unevenness is less observable while the soft texture is maintained.

### <Definition of Terms in the Description>

The following terms in the description shall have the following means unless otherwise specified in the description.

- The "front body" and "back body" refer to the portions on the front side and the back side, respectively, of the center in the front-back direction of the underpants-type disposable diaper. The crotch portion refers to the area in the front-back direction including the center in the front-bac direction of the underpants-type disposable diaper and, when the absorbent body has a narrower portion, refers to the area in the front-back direction including the narrower portion.

- The "front-back direction" refers to the direction shown by reference sign LD in the drawings (longitudinal direction), whereas the "width direction" refers to the direction shown by reference sign WD in the drawings (right-left direction). The front-back direction and the width direction are orthogonal to each other.

- The "elongation at elastic limit" means the elongation at elastic limit in the stretchable direction ED (in other words, the state in which the first sheet layer and the second sheet layer are completely spread), and refers to the length at the elastic limit expressed in percentage based on the natural length being 100%.

- The "area rate" means the rate of the objective portion occupying the unit area, and refers to the value expressed in percentage obtained by dividing the sum of the areas of objective portions (e.g., joining portions 40, openings of the joining holes 31, vent holes) in an objective region (e.g., stretchable regions 80, non-stretchable regions 70) by the area of the said objective region. In particular, the "area rate" in the region having the stretchable structure refers to the area rate in the state stretched in the stretchable direction ED to the elastic limit. When multiple objective portions are provided at intervals, the area rate is preferably determined by setting the objective region to a size containing ten or more of the objective portions.

- The "stretch rate" refers to a value based on the natural length being 100%. For example, 200% stretch rate means the same as the two-fold stretch.

- The "fineness" is determined as follows. A sample or a test piece is preliminarily dried, and left in a testing room or testing apparatus under the standard conditions (the testing location is at a temperature of 23 ± 1 °C and relative humidity of 50 ± 2%) until it is at constant mass. The preliminary drying refers to making of a sample or a test piece into constant mass in the environment at a temperature of 100 °C. Incidentally, fibers having an official regain of 0.0% do not have to be subjected to the preliminary drying. A sample of 100 mm ×100 mm in size is cut out of the test piece in constant mass using a sampling template (100 mm ×100 mm). The weight of the sample is measured and multiplied by 100 to determine the weight per one square meter, which is taken as the fineness.

- The "thickness" other than the above is automatically determined using automatic thickness measurement equipment (KES-G5 handy compression testing program) under the load of 0.098 N/cm² and the compression area of 2 cm².

- The "tensile strength" and the "tensile elongation (breaking elongation)" refer to the values determined according to JIS K7127: 1999 "Plastics-Determination of tensile properties" with the initial chuck clearance (gauge line distance) of 50 mm and the tension rate of 300 mm/min, except that the test piece is in a rectangular shape of 35 mm wide by 80 mm long. As a tensile testing machine, for example, AUTOGRAPH AGS-G100N manufactured by SHIMADZU CORPORATION may be used.

- The "stretching stress" refers to the tensile stress (N / 35 mm) determined in stretching in the elastic region by the tensile test according to JIS K7157: 1999 "Plastics-Determination of tensile properties" with the initial chuck clearance (gauge line distance) of 50 mm and the tension rate of 300 mm/min, and the degree of stretching may suitably be decided depending on the sample to be tested. The test piece is preferably in a rectangular shape of 35 mm wide by 80 mm long or longer, but when a test piece of 35 mm wide cannot be cut out, a test piece is cut out at a possible width and the measured value is converted to the value corresponding to the width of 35 mm. Further, even when the objective region is too small to take a test piece of sufficient size, for the purpose of comparing the magnitude of the stretching stress, comparison is still possible as long as the test pieces of the same size, even if reasonably small, are used. As a tensile testing machine, for example, AUTOGRAPH AGS-G100N manufactured by SHIMADZU CORPORATION may be used.

The L* value, the a* value, and the b* value of the "CIELAB" may be determined by, for example, NF555 manufactured by NIPPON DENSHOKU INDUSTRIES CO., LTD.

- The "spread state" means the state of flat spread without contraction or slack.

- The dimensions of each portion mean the dimensions in the spread state, not in the natural length state, unless otherwise specified. In particular, the dimensions of the joining portions are the dimensions in the state in which the article is spread to the limit (state before the first sheet layer and the second sheet layer are fractured), and substantially identical with the dimensions of the joining portion pattern on the anvil roll.

- A test or a measurement is performed in a testing room or testing apparatus under the standard conditions (the testing location is at a temperature of 23 ± 1 °C and relative humidity of 50 ± 2%) unless the environmental conditions are otherwise specified.

### Industrial Applicability

The present invention may be applied to general disposable wearable articles, including, in addition to the underpants-type disposable diapers of the embodiments discussed above, various disposable diapers, such as of tape type or pad type, sanitary napkins, disposable wearable articles for swimming or playing with water.

### Description of Reference Signs

10: inner member; 10B: inner-outer fixed region; 11: top sheet; 12: liquid impervious sheet; 13: absorbent body; 13N: narrower portion; 14: wrapping sheet; 17: no-absorbent side portion; 20: outer member; 20A: first sheet layer; 20B: second sheet layer; 20C: folded portion; 20X: elastic sheet stretchable structure; 21: side seal portion; 23: waist end portion; 24: waist portion elastic member; 25F, 25f: ridge; 29: around-leg line; 30: elastic sheet; 31: joining hole; 40, 40A, 40B: joining portion (first joining portion); 41: second joining portion; 42: third joining portion; 43: fourth joining portion; 50, 51: cover sheet; 70: non-stretchable region; 80: stretchable region; 82: edge stretchable portion; 90: three-dimensional gather; 93: laid-down portion; 94: free portion; 95: gather sheet; 96: gather elastic member; B: back body; ED: stretchable direction; F: front body; L: intermediate portion; XD: orthogonal direction; LD: front-back direction; T: lower torso portion; sf: small pleat; bf: large pleat; 100: colored nonwoven fabric; 101: outermost layer; 110: colored layer; 120: base sheet

## Claims

1. A disposable wearable article comprising:
colored nonwoven fabric having an exposed portion, and
a base sheet adjacent to an inner side of the colored nonwoven fabric,
wherein the colored nonwoven fabric is a composite nonwoven fabric having a plurality of layers laid one on top of another,
wherein the colored nonwoven fabric has, in addition to an outermost layer, a colored layer colored in a color of a first lightness other than white,
wherein the outermost layer of the colored nonwoven fabric has a color of a second lightness higher than the first lightness, and
wherein an external surface of the base sheet has a color of a third lightness higher than the first lightness.

2. The disposable wearable article according to claim 1, wherein the color of the first lightness other than white has a CIELAB L* value of 20 to 90, and an absolute value of at least one of a* and b* values is 0 to 40,
wherein the color of the second lightness and the color of the third lightness each has a CIELAB L* value of 60 to 100, and
wherein a color difference **ΔE** between the colors of the second lightness and the third lightness is 30 or less.

3. The disposable wearable article according to claim 1 or 2, wherein the colored nonwoven fabric is a composite nonwoven fabric of 2 to 4 layers having a fineness of 1.5 to 5.0 dtex and a basis weight of 10 to 20 g/m².

4. The disposable wearable article according to any one of claims 1 to 3,
wherein the disposable wearable article has an elastic sheet stretchable structure comprising a first sheet layer, a second sheet layer facing to the first sheet layer and exposed to an external surface of the article, and an elastic sheet laid therebetween,
wherein the first sheet layer and the second sheet layer are joined through joining holes penetrating the elastic sheet at multiple joining portions arranged at intervals,
wherein the colored nonwoven fabric is the second sheet layer, and
wherein the base sheet is the elastic sheet.

5. The disposable wearable article according to any one of claims 1 to 4, further comprising an absorbent body, a liquid impervious sheet covering a back surface side of the absorbent body, and outer nonwoven fabric covering a back surface side of the liquid impervious sheet,
wherein the colored nonwoven fabric is the outer nonwoven fabric, and
wherein the base sheet is the liquid impervious sheet.
